# EUROPEAN PATENT SPECIFICATION

(11) **EP 1 256 052 B1**
(45) Date of publication and mention of the grant of the patent: **09.02.2005**
(21) Application number: 00952336.6
(22) Date of filing: 31.07.2000
(51) Int. Cl.: G06F 7/00

(54) **METHODOLOGY FOR IDENTIFYING PLANTS AND OTHER ORGANISMS HAVING TRAITS DIFFERING FROM A NORMAL POPULATION**
METHODOLOGIE UM PFLANZEN UND ANDERE ORGANISMEN MIT VON DER NORMALEN PROBE ABWEICHENDEN CHARAKTERISTIKEN ZU IDENTIFIZIEREN
PROCEDE D'IDENTIFICATION DE PLANTES ET D'AUTRES ORGANISMES PRESENTANT DES CARACTERISTIQUES DIFFERENTES D'UNE POPULATION NORMALE

(30) Priority: 03.08.1999 US 146871 P; 22.06.2000 US 213345 P
(43) Date of publication of application: 13.11.2002
(73) Proprietor: Paradigm Genetics, Inc., Research Triangle Park, NC 27709-4528 (US)
(72) Inventor: BOYES, Douglas, C., Chapel Hill, NC 27514 (US); GORLACH, Jorn, Durham, NC 27707-5647 (US); BARRETT, Thomas, H., Raleigh, NC 27612 (US); HOFFMAN, Neil, Chapel Hill, NC 27514 (US); MCCASKILL, Amy, J., Apex NC 27502 (US); DAVIS, Keith, R., Durham, NC 27707 (US)
(74) Representative: Kensett, John Hinton
(86) International application number: PCT/US2000/020878
(87) International publication number: WO 2001/009711

(56) References cited:
- WO-A-97/14106
- CHARMET ET AL: 'Agronomic evaluation of a collection of french perennial rygrass..' AGRONOMIE vol. 19, no. 10, PARIS, pages 807 - 824
- MAUGHAN P.J. ET AL: 'Molecual-marker analysis of seed-weight ...' THEORETICAL AND APPLIED GENETICS vol. 93, no. 4, pages 574 - 579

## Description

### FIELD OF THE INVENTION

The present invention relates to a method, computer readable media, a computer system, a computer, computer memory and a high-throughput system for determining whether a test candidate organism has at least one trait (phenotypic or genetically based) that differs from a control population.

### BACKGROUND OF THE INVENTION

Biotechnology is the pragmatic combination of science and technology to make use of our knowledge of living systems for practical applications. This includes a wide variety of applied biological sciences, but can also include aspects of chemistry, chemical technology, engineering, and specialist disciplines of specific industries such as pharmaceuticals, environmental treatment and agricultural industries. Advances in the science of sequencing portions of genes made of DNA (deoxyribonucleic acid) have enabled scientists to generate huge amounts of data about the DNA sequences for a given organism, such as plants, mice, etc. However, increasingly it is clear that automated sequencers and high-throughput mapping programs can generate genetic data far faster than scientists can understand it or turn it into practical results. That is, merely possessing the DNA sequence of a gene still does not tell the scientist the function or utility of that gene. To determine the function of gene, another scientific discipline has emerged known as functional genomics, which attempts to assign function to a gene, in part, by correlating phenotypic manifestations to genetic sequences.

Previous studies have attempted to determine the function of an individual gene at a particular stage of an organism's growth and development. However, these studies tend to be limited in that they examine only a few of an organism's traits. Further, such studies tend to focus on only a particular growth stage of an organism. The availability of the nearly complete Arabidopsis genomic sequence has paved the way for the development of numerous reverse genetic approaches for the determination of gene function. In most cases, the identification of mutations in the gene of interest may be a straightforward, if sometimes laborious process (Krysan, P.J., Young, J.C. & Sussman, M.R. T-DNA as an insertional mutagen. *Plant Cell* 11, 2283-2290 (1999). Charmet et al, Agronomie,10, p.807 - 824 (1990) disclose a method of agronomic evaluation of a collection of French perennial ryegrass populations by multivariate classification of genotype x environment interactions. However, difficulties often arise in the identification of a phenotype that can be associated with the mutation. This is particularly true in plants, where genes often exist as members of multigene families that exhibit redundant or highly specialized functions. Homology searching, microarray-based analysis of gene expression and metabolic studies may provide some information regarding gene function. In most cases, however, a complete understanding of a gene's function will only be realized when such information can be associated with a phenotype at the organismal level. Many of these phenotypes may be manifested as subtle changes in growth or development, underscoring the need for a sensitive and robust methodology for their detection. There is a need for methodologies that can systematically generate a phenotypic profile for an organism throughout its various stages of growth and development. In this way, test candidate organisms can be identified that have at least one trait that differs from a control population.

### SUMMARY OF THE INVENTION

Our approach to this problem has been to develop an extensive phenotypic analysis process for capturing data describing growth and development over most of or the entire the life of an organism, such as a plant. Single gene mutations or altered environmental conditions may affect any number of traits, resulting in morphological changes and/or altered timing of development. Morphological changes can often be readily identified and recorded outside the context of extensive temporal or growth stage information. In contrast, mutations that result in altered developmental progression can be recorded only if a temporal component is included in the analysis. Such time-course analyses are inevitably resource intensive and must be restricted in scope to allow the analysis of many samples in parallel. We have addressed this issue by developing a method for phenotyping organisms, such as plants, based on a series of defined growth stages. The growth stages serve both as developmental landmarks and as triggers for the collection of morphological data that is of interest at specific stages of development.

Accordingly, in one embodiment, the present invention is directed towards a method for identifying a test candidate organism that has at least one trait that differs from a control population, the method comprising:
a) establishing the stages of growth for an individual control organism or for a population of control organisms and the criteria for determination thereof;
b) programming a computer to identify what stage of growth an individual organism is at or a population of organisms is at, based upon the criteria established in step a);
c) establishing the traits of an organism that may be manifested or associated with said growth stages and the criteria for measurement thereof;
d) programming a computer to identify what additional traits of an organism are to be measured according to the criteria established in step c);
e) growing control organisms and at least one test candidate organism;
f) providing measurements of growth stage determining traits from said control organisms and said test candidate organism to said computer that will enable the computer to specify
   i) what stage of growth an organism is at; and
   ii) what traits of the organism are to be measured at that growth stage;
g) measuring those traits specified by the computer for control and test candidate organisms;
h) providing data on the measurements of those traits to the computer;
i) optionally, repeating steps f), g) and h) for additional growth stages to be measured; and
j) identifying those test candidate plants having at least one trait that differs from the control population based upon said measured traits of growth stage and/or traits that may be manifested or associated with at least one growth stage.

In another embodiment, the present invention is directed towards a computerized method for identifying a test candidate organism that has at least one trait that differs from a control population, the method comprising:
a) obtaining measurements of traits that determine a growth stage for said control population and said test candidate organism;
b) optionally, determining what stage of growth an organism is at based upon measurements obtained in step a);
c) determining what additional traits of the organism are to be measured at that growth stage;
d) obtaining measurements for traits as determined in step c) that are manifested or associated with that growth stage for said control and said test candidate organisms;
e) optionally, repeating steps a), b), c) and d) for additional growth stages to be measured; and
f) optionally, identifying those test candidate organisms having at least one trait that differs from the control population based upon said measured traits of growth stage and/or traits that may be manifested or associated with at least one growth stage.

In another embodiment, the present invention is directed towards a computer-readable medium containing instructions for a method to enable the identification of a test candidate organism having at least one trait that differs from a control population, the method comprising:
a) obtaining measurements of traits that determine a growth stage for said control population and said test candidate organism;
b) optionally, determining what stage of growth an organism is at based upon measurements obtained in step a);
c) determining what additional traits of the organism are to be measured at that growth stage;
d) obtaining measurements for traits as determined in step c) that are manifested or associated with that growth stage for said control and said test candidate organisms;
e) optionally, repeating steps a), b), c) and d) for additional growth stages to be measured; and
f) optionally, identifying those test candidate organisms having at least one trait that differs from the control population based upon said measured traits of growth stage and/or traits that may be manifested or associated with at least one growth stage.

In another embodiment, the present invention is directed towards a computer-readable medium whose contents transform a computer system into a system for identifying a test candidate organism that has at least one trait that differs from a control population, the medium comprising:
a) a subsystem which receives measurements of growth stage determining traits;
b) an optional subsystem which determines what stage of growth an organism is at based upon measurements received in subsystem a);
c) a subsystem which specifies what traits of the organism are to be measured at that growth stage;
d) a subsystem which receives measurements of traits of the organism to be measured at a growth stage for said control and said test candidate organisms;
e) an optional subsystem which can repeat steps a), b), c) and d) for additional growth stages to be measured; and
f) an optional subsystem which can identify those test candidate organisms having at least one trait that differs from the control population based upon said measured traits of growth stage and/or traits that may be manifested or associated with at least one growth stage.

In another embodiment, the present invention is directed toward a computer system programmed to identify a test candidate organism that has at least one trait that differs from a control population, the system comprising:
a) a subsystem which receives measurements of growth stage determining traits;
b) an optional subsystem which determines what stage of growth an organism is at based upon measurements received in subsystem a);
c) a subsystem which specifies what traits of the organism are to be measured at that growth stage;
d) a subsystem which receives measurements of traits of the organism to be measured at a growth stage for said control and said test candidate organisms;
e) an optional subsystem which can repeat steps a), b), c) and d) for additional growth stages to be measured; and
f) an optional subsystem which can identify those test candidate organisms having at least one trait that differs from the control population based upon said measured traits of growth stage and/or traits that may be manifested or associated with at least one growth stage.
In another embodiment, the present invention is directed towards a computer memory containing a data structure for storing data relating to traits of control and test candidate organisms comprising:
a) a stage of growth table containing an entry of traits for each organism each entry containing a pointer to linked to,
b) a list of measurements table specifying what traits of an organism are to be measured at that growth stage; and
c) an optional counter which can repeat steps a) and b) for additional growth stages to be measured.

In another embodiment, the present invention is directed towards a method for identifying a test candidate organism that has at least one trait that differs from a control population, the method comprising:
a) obtaining measurements of traits that determine a growth stage for said control population and said test candidate organism;
b) determining what stage of growth an organism is at based upon measurements obtained in step a);
c) determining what additional traits of the organism are to be measured at that growth stage;
d) obtaining measurements for traits as determined in step c) that are manifested or associated with that growth stage for said control and said test candidate organisms;
e) optionally, repeating steps a), b), c) and d) for additional growth stages to be measured; and
f) identifying those test candidate organisms having at least one trait that differs from the control population based upon said measured traits of growth stage and/or traits that may be manifested or associated with at least one growth stage.

In the above method(s), computerized method, computer-readable medium(s) and computers system the organism can be a plant, such as a dicot or a monocot plant. Where the organism is a dicot plant, the growth stage can be one or more of the following: germination, leaf production, cotyledon growth, rosette growth, infloresence emergence, flowering, fruit development, fruit ripening, senenscence, or combinations thereof. Traits of the organism that can be measured can be one or more of the following characteristics: developmental, morphological, visual, physiological, molecular, biochemical in nature or combinations thereof. Also, measurements of traits can be collected using one or more of the following: macroscopic photography, physical measurements, dissection, color quantification, microscopic photography, weight determination, image analysis, fluorescence spectroscopy measurements or combinations thereof.

In another embodiment, the present invention is directed towards a method that provides for the reproducible collection, storage and analysis of phenotypic data that describe an organism's development and it's ability to respond to environmental change. In this embodiment, the method comprises a biological component (the scientific method utilized to collect phenotypic data) and a computer-based component (the software and hardware that control the workflow and data collection processes). In the described embodiment, plant phenotypes are quantified through an analysis of growth, development and physiology.

One advantage of the present invention is that it provides a means for developing a quantitative high-throughput analysis platform to define phenotypes resulting from genetic or environmental variation. Genetic variations can be those occurring naturally in a population or can result from chemical induced mutations (ethylmethane sulfonate or ems) or biological induced mutations (DNA insertions by bacteria or viruses). Environmental variations can be those induced by biotic stress (pathogen or disease) or abiotic stress (drought, extreme temperatures, sub-optimal nutrition, extreme light conditions, etc.). High throughput analyses allow for the rapid and efficient identification of genes having agronomic, pharmaceutical, nutritional and industrial importance.

Another advantage of the present invention is that it allows one to integrate data representing traits over the entire life cycle of an organism, such as a plant, into a single data set. Such integration enables one to perform multivariate analysis in order to identify correlative relationships among highly diverse traits at different stages of growth, thereby fostering identification of easily measured and highly reproducible traits that can serve as surrogates for more difficult to measure or more variable traits, such as yield. Ultimately, these surrogate traits may be tested for validity in crop species and developed as transgenic products or as tools to aid in conventional breeding programs.

Another advantage of the present invention is that it allows one to use phenotypic fingerprinting. Phenotypic fingerprinting refers to the grouping of traits that correlate with a particular phenotype, such as plant drought resistance. For example, phenotypic fingerprinting could correlate one, two, three or more traits associated with the phenotype of drought resistance in plants that could be used as markers. Once such drought resistant traits have been identified, other plants can be evaluated for drought resistance at an early stage of the plant development. Alternatively, phenotypic fingerprinting can also refer to the grouping of traits that correlate with a particular mode-of-action in response to an environmental stress, e.g. herbicide application, nutritional deficiency or extremes in temperatures.

Another advantage of the present invention is that the growth stages and data collection methodology presented here can serve as a powerful means to unify the collection of phenotypic data. Reporting the growth stage from which data were obtained can provide an explicit developmental context for comparative purposes and enhance its value for future *in silico* investigations.

### BRIEF DESCRIPTION OF THE FIGURES

FIG. 1 is a diagram depicting the core elements of the phenotypic analysis platform.
Fig. 1A is a graph depicting representative data taken from the analysis of wild-type Col-0 plants.
Fig. 1B is a graph depicting a principal growth stage 1, in which the number of rosette leaves is the growth-stage-determining trait.
Fig. 1C is a graph depicting principal stages 3, defined as a percentage of the final value of the trait being measured, in this case, maximum rosette radius.
Fig 1D is a graph depicting stem length over time.
FIG. 2 provides a generic flow chart of the process used to evaluate traits for inclusion in the phenotypic analysis platform.
Fig. 2A provides a graph depicting a summary of growth stage progression for wild type (i.e. control) and five mutant lines.
Fig. 2B provides a graph comparing silique area and the number of seeds per silique between adg1-1 mutants and the control.
Fig. 2C provides a graph comparing silique number and the yield per plant between adg1-1 mutants and the control.
FIG. 3 provides an example of sample types and relationships.
FIG. 4 illustrates the logic used to schedule high throughput phenotypic data collection.
FIG. 5A & B are examples of a prototype graphical data collection interface.
FIG. 6 illustrates an experimental protocol for the phenotypic analysis of Arabidopsis plants grown on agar plates.
FIG. 7 illustrates an experimental protocol for the phenotypic analysis of Arabidopsis plants grown on soil.
FIG. 8 provides a generic flow chart depicting a method for identifying a test candidate organism that has at least one trait that differs from a control population.

### DETAILED DECRIPTION OF THE INVENTION

As used herein, the terms "comprising," "by" and "comprising the steps of" are considered to have substantially the same meanings.

As used throughout this specification, the following definitions apply for purposes of the present invention:
computer: a device that computes, especially a programmable electronic machine that performs high-speed mathematical or logical operations or that assembles, stores, correlates, or otherwise processes information.
computer memory: component of a computer that stores instructions and data for rapid access by the processing units.
computer readable medium (or media): a permanent or semi-permanent and transportable device that stores instructions and/or data used by a computer. Examples include but are not limited to magnetic tape, optical disk (such as CD-ROMs), semiconductor memory (Read Only Memory - ROM), magnetic diskette and hybrids of these categories such a magnetic/optical storage media.
computer system: a collection of devices and software components that work in a coordinated fashion to process and store information.
control population: a population of organisms that are used as a standard of comparison. A control population establishes a test or experiment established as a check of other experiments, performed by maintaining substantially identical conditions except for the one varied factor, whose causal significance can thus be inferred. The factor can be a genetic or environmental influence imparted to or on the test candidate organism. A control population is sometimes referred to as a "normal" population.
dicots or dicotyledonous: plants having two seed leaves or cotyledons including *Arabidopsis,* tomatoes, soybeans, cotton, oilseed rape, flax, sugar beet, sunflower, potato, tobacco, lettuce, peas, beans, alfalfa and the like.
high-throughput system: a set of devices and procedures designed to process a large number of items in a short period of time with great efficiency and typically a significant amount of automation.
measurements: refers to qualitative and quantitative data collected or taken.
monocots or monocotyledonous: plants having one cotyledon such as rice, maize, wheat, sorghum and the like.
mutant: an organism bearing a mutant gene that expresses itself in the phenotype of the organism.
organism: a biological individual.
phenotype: the observable properties of an organism, produced by the genotype in conjunction with the environment.
population: a group of organisms sharing a common gene pool.
programming: creating and/or modifying software for a computer.
software: the programs, routines, and/or symbolic languages that control and/or provide instructions to the hardware, direct its operation and result in one or more desired outcomes.
stage of growth: a specific point in the development of an organism that is reached upon meeting pre-determined criteria related to growth and development.
subsystem: hardware and/or software components that perform a part or a portion of the functions of a greater computer system.
test candidate organism: organism being examined for having at least one trait that differs from the control population.
trait - any detectable or measurable phenotypic characteristic of an organism, including the developmental, morphological, visual, physiological, molecular and/or biochemical characteristic of the organism. The measure of such traits may be quantitative and/or qualitative.
wild-type - the most frequently observed phenotype or the one designated as normal or control.

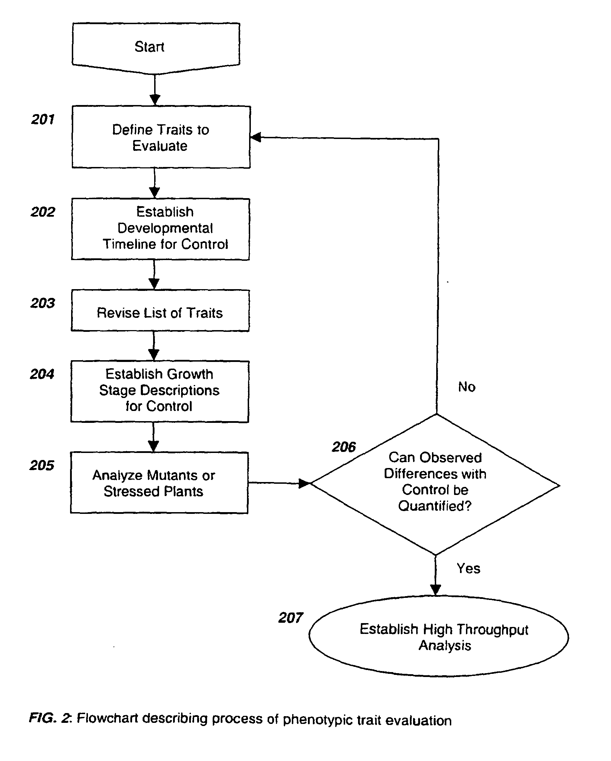

Historically, biological experiments have been designed to address a targeted research question. However, the nature of genome-scale research, coupled with the rapid development of databases to store and mine the resulting data, now makes it possible for the same data to be mined by scientists with completely different research objectives. Since the data used to populate these databases will often be integrated from many different sources, the ultimate success of the mining process is dependent on the uniformity of the methods used for its collection. In this light, we have developed a standardized process for the collection of phenotypic data that is built upon a series of growth stage definitions. The growth stages serve both as developmental landmarks and as triggers for the collection of detailed morphological data. The process is generic and can be applied to any organism for which growth stages have been defined. Using this method, we have established a data set representative of wild-type Col-0 plants grown under standardized environmental conditions and validated our ability to detect novel phenotypes through the characterization of single gene mutations (Fig. 2A). The same process can be used for the detection of phenotypic alterations resulting from more divergent genetic backgrounds (*e.g*., different ecotypes) as well as those resulting from the introduction of biotic or abiotic stress.

Developmental growth stages have been described at the organismal level for a variety of experimental models, including *D. melanogaster* (Hartenstein, V. *Atlas of Drosophila morphology and development.* (Cold Spring Harbor Laboratory Press, Planview, NY; (1993) and *C. elegans* (Wilkins, A.S. *Genetic analysis of animal development.* (Wiley-Liss, Inc., New York, NY; (1993). However, with the exception of definitions describing the phases of specific organs or tissue types (Smyth, D.R., Bowman, J.L. & Meyerowitz, E.M. Early flower development in *Arabidopsis. Plant Cell* 2, 755-767 (1990), very little unification in the stages of *Arabidopsis* growth and development has been achieved. Growth stage definitions for other plants have been developed, and are utilized routinely in the breeding industry. One such example is the BBCH scale, which was proposed to provide a generic nomenclature for the assignment of growth stages in crop plants and weeds (Lancashire, P.D., Bleiholder, H., v.d. Boom, T., Langeluddeke, P., Stauss, R., Weber, E. & Witzenberger, A. A uniform decimal code for growth stages of crops and weeds. *Ann. Appl*. *Biol*. 119, 561-601 (1991). We have utilized the BBCH scale as a basis from which to define a series of growth stages for use in the phenotypic analysis of *Arabidopsis*. The analysis of wild-type development presented here illustrates a framework methodology for identifying and interpreting phenotypic differences in plants resulting from genetic variation and/or the impact of environmental stress. This phenotypic analysis platform, optionally in conjunction with metabolic and gene expression profiling analyses conducted in parallel, can provide a robust method for the high throughput functional analysis of plant genes.

As the genomic sequencing project of living species such as *Arabidopsis*, Saccharomyces and others nears completion the next challenge is the large-scale determination of gene function. In *Arabidopsis,* this challenge has been addressed through the development of a high throughput phenotypic analysis platform for capturing data describing growth and development over the entire life of the plant. The platform is based on a series of defined growth stages that serve both as developmental landmarks and as triggers for the collection of morphological data that is of interest at specific stages of development. We propose these growth stages as a means by which phenotypic data of all types can be collected and compared within the *Arabidopsis* research community. When combined with parallel processes for metabolic and gene expression profiling, this platform serves as a core technology in the high throughput determination of gene function.

Other types of organisms amenable to analysis with the present invention includes, but is not limited to fungi (such as plant pathogenic filamentous fungi), insects, nematodes and mammals (such a mice). Growth stages and traits which are manifested or associated with that growth stage (in parentheses) for a fungi can include spores (spore size, shape and color), germination (percent germination), appressorium formation (length of germ tube, appressorium size, shape and color), hyphal growth (hyphal area, average diameter), haustoria formation (haustoria density per unit leaf area), sporangiophore production (hyphal area, average diameter), spore release (Number of spores produced per unit leaf area) and combinations thereof.

Growth stages and traits which are manifested or associated with a growth stage for an insect can include egg (egg size, shape and color), larva (larva weight at different instars), pupa (pupa size, shape and color), adult (size of segments and appendages) and combinations thereof.

Growth stages and traits which are manifested or associated with a growth stage for a nematode can include egg (egg size, shape and color), first larval stage-L1 (weight and length), second larval stage-L2 (weight and length), third larval stage-L3 (weight and length), fourth larval stage (weight and length), adult (weight, length and mobillity) and combinations thereof.

Growth stages and traits which are manifested or associated with a growth stage for a mouse can include one-cell egg, dividing egg, morula, blastocyst (inner cell mass apparent), Implantation and formation of egg cylinder (ectoplacental cone appears, enlarged epiblast), Late head fold (LHF), Turning of the embryo (1st branchial arch has maxillaryand mandibular components, 2nd arch present, Absent 3rd branchial arch), Earliest sign of fingers (splayed-out), Anterior footplate indented (elbow and wrist identifiable, 5 rows of whiskers, umbilical hernia now clearly apparent, hair follicles, fingers separate distally), Fingers separate distally(only indentations between digits of the posterior footplate, long bones of limbs present, hair follicles in pectoral, pelvic and trunk regions, absent open eyelids), Fingers & Toes separate (hair follicles also in cephalic region but not at periphery of vibrissae, eyelids open, absent nail primordia, fingers 2-5 parallel), Reposition of umbilical hernia, eyelids closing, fingers, 2-5 are parallel, nail primordia visible on toes, Absent wrinkled skin, fingers & toes joined together, Newborn Mouse Postnatal development or combinations thereof.

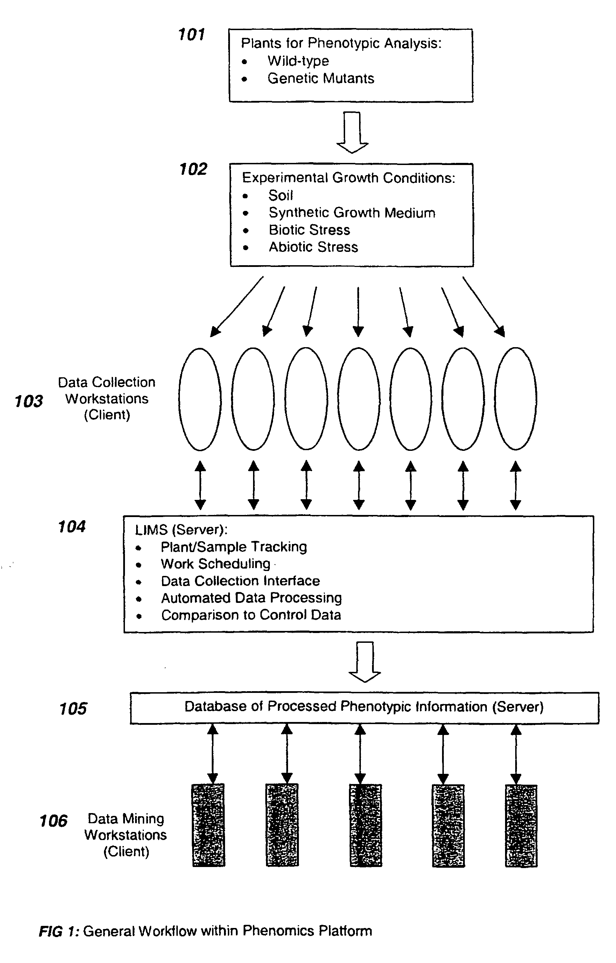

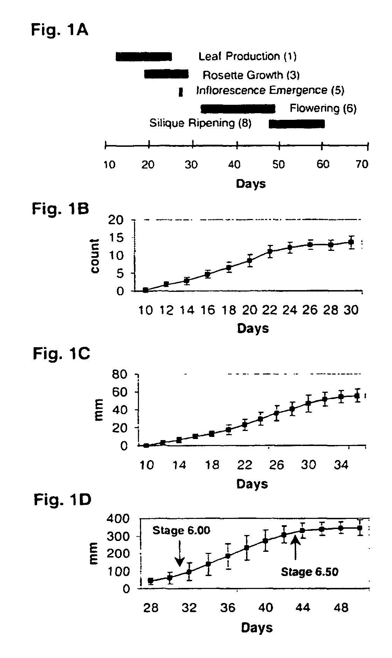

The present invention comprises six core elements as depicted in **FIG. 1.** The organism for study ***101*** may be either a naturally occurring species or accession, or an experimentally modified derivative thereof. In one possible embodiment, data describing the phenotype of a genetically modified experimental organism is compared to that of other experimental organisms and to an unmodified ("wild type") control. A comprehensive phenotypic analysis platform hinges on the analysis of plants grown under a wide range of the conditions ***102***. For instance, many stages of the plant life cycle, such as pollination and fertilization, are best studied on soil grown plants. However, characterizing the early stages of plant development and the identification of seedling lethal mutations is difficult in soil. For these analyses plants grown *in vitro* on solid agar medium are utilized. Such assays also allow complete control over the growth media composition, permitting the effects of nutritional deficiencies and chemical stress treatments to be evaluated. Hydroponic growth systems allow similar control of the growth media, while at the same time allowing plants to grow in an open-air environment. Additional phenotypic information can be obtained by growing plants under extremes in temperature, humidity or light or by treating plants with biotic (*e*.*g*., pathogens) or abiotic (*e*.*g*., herbicides) agents. In all cases the growth conditions and treatments must be consistent and highly reproducible to foster meaningful comparisons among plants grown and/or treated at different times.

Phenotypic data is collected at a series of experimental workstations ***103***. Each workstation is manned by one to two people and is designed to perform a specific data collection function. This modular design allows for efficient parallel processing of numerous plant samples. Plants and plant-derived samples move from station to station with data collection occurring at each step along the way.
Examples of workstations to address plant growth, development and physiology include:
1. Macroscopic Photography
   Process:
   Take overhead, frontal and oblique digital images of whole plants in pots
   Take overhead images of dissected plant organs and plants grown on plates
   Export images to software package for analysis

   Experimental Equipment:
   Digital cameras mounted in fixed positions (overhead, frontal)
   Copy stand with lighting
   Base board with hole to position pot
   Back drop
   Built-in length standards

   Computer Requirements:
   2 NT work stations (one for each camera)
   One bar code reader per computer

   Space: ∼6 feet of bench (copy stand should be placed on low bench or short table for easy access)
2. Physical Measurement (Manual)
   Process:
   Take non-destructive measurements from plants in a flat
   Enter the data directly into the database

   Experimental Equipment:
   Micrometer
   Ruler
   Calipers

   Computer Requirements:
   One NT workstation with barcode reader (eventually may need 2 computers here)

   Space: 2 sections of low bench with kneeholes
3. Plant Dissection and Color Quantitation
   Process:
   Dissect plants for detailed photographic and destructive measurements
   Remove leaves and other organs, take spectral reflectance measurements and export data to database
   Arrange leaves and other organs under glass plate for photography

   Experimental Equipment:
   Scalpel, forceps
   Color spectrophotometer
   Non-glare glass plates
   Filter paper, tape

   Information Technology Requirements:
   One NT workstation (with barcode reader) to interface with color spectrophotometer and run associated software

   Space: 6-8 feet of bench space with kneehole
4. Microscopic Plant Photography
   Process:
   Capture digital image of dissected plant parts
   Export images to analysis software

   Experimental Equipment:
   Dissecting microscope & camera for taking pictures of flowers, seeds, trichomes, etc.
   Compound microscope & camera for taking pictures of pollen, histological sections, etc.

   Computer Requirements:
   Two NT workstations with barcode readers to operate cameras

   Space: 8-10 feet of low bench space with kneeholes
5. Weigh Station (weight determination)
   Process:
   Obtain fresh weight of dissected organs
   Dry tissue overnight in oven
   Obtain dry weight of organs

   Experimental Equipment:
   Analytical balance
   Drying oven (50C)

   Computer Requirements:
   One NT workstation (with barcode reader) interfaced with balance for direct data entry

   Space: 8 ft. of high bench with knee hole
6. Mature Plant Harvest/ Seed Analysis
   Process:
   Harvest seed from mature plants
   Prepare seed for photography, volume and weight analysis

   Experimental Equipment:
   Screens for harvesting seed
   Biohazard container

   Computer Requirements:
   One NT workstation with barcode reader

   Space: Bench with one kneehole; located next to weigh station
7. Image Analysis
   Process:
   Download images and extract measurements
   Export measurements to database
   Archive images

   Experimental Equipment:
   None

   Computer Requirements:
   Image analysis software running on dedicated PC

   Space: Desk or counter space with kneeholes for 2 computers
8. Fluorescence Spectroscopy Measurements
   Process:
   Couple variable wavelength excitation with a series of filters to observe a variety of distinct fluorescence emission spectra
   Examples of biological processes that can be addressed include photosynthetic capacity and accumulation of fluorescent metabolites

Each workstation is outfitted with at least one Windows NT computer networked to a central server. This computer allows access to the client-server Laboratory Information Management System (LIMS) software ***104***. The LIMS controls all aspects of plant and plant-derived sample tracking, workflow scheduling and also provides the data collection interface. In addition the LIMS provides preliminary data processing and comparison functions prior to passing the data to the central phenotypic database ***105*** where it is accessible for detailed mining ***106***. The scope of the current invention is restricted to the biological analyses and computer systems that make up the phenotypic data collection and storage platform. Downstream computer systems required for data mining are not within the scope of the invention and will not be described further.

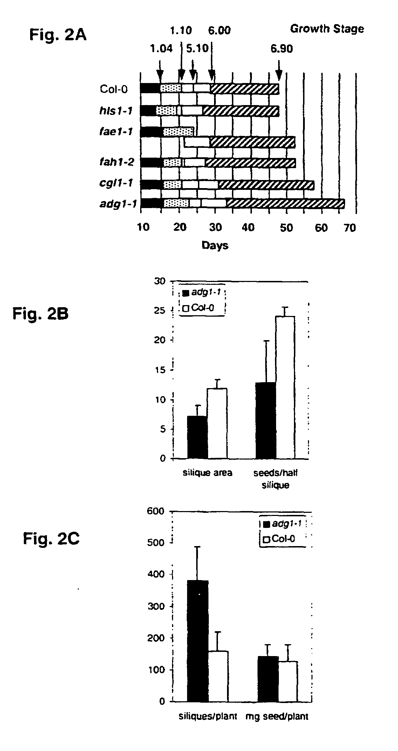

The process utilized to evaluate biological characters for inclusion in the phenotypic analysis platform is depicted in **FIG. 2**. The process initiates with the generation of a list of traits or characters to evaluate ***201***. Depending on the experimental design, traits for evaluation may number in the tens or hundreds and are chosen to quantitatively describe phenotypes that are well understood and of known biological significance as well as those that are poorly characterized with unknown biological significance. Each trait or character must be evaluated for biological variation prior to inclusion in the platform. Typically, this is accomplished by conducting a thorough analysis of the traits in control populations ***202.*** Many traits vary over, or are dependent on the developmental progression of the organism. Biological variation in these traits is likely to change over the course of development. Thus, it is important to establish a detailed developmental timeline to determine the optimal time to measure each trait of interest. For example, in *Arabidopsis* the rate of stem elongation is very rapid during the initial reproductive phase of development. During this time the biological variation in stem length is very large. However, later in development the rate of elongation begins to slow and the variation is reduced substantially. Thus, stem length measurements are most meaningful when taken after the initial phase of rapid growth has concluded. Other traits in *Arabidopsis,* such as the distance between the base of the stem and the first stem branch exhibit a standard deviation that is greater than 50% of the average. This level of variation makes meaningful comparisons between control and experimental data very difficult. Therefore, during the process of revising the list of traits to be analyzed ***203,*** those falling into this category are eliminated.

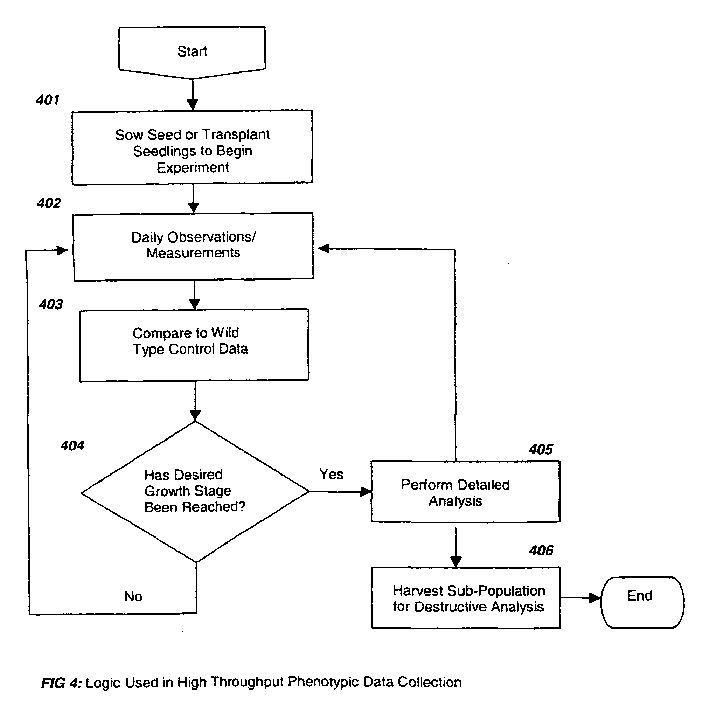

While control plants grown under constant conditions develop according to a standard timeline, genetic mutations or changes in the environment of a plant can have profound affects on the timing of development. Because of this, comparison of data collected from plants of similar age will not always result in comparison of similar growth stage. Indeed, it is likely that such comparisons will result in extremes such as data from pre-flowering plants being compared with that obtained from flowering plants. This situation can be avoided by using control phenotypic data to establish general growth stage descriptions ***204*** and only comparing data obtained from plants of similar growth stage. This concept is presented in greater detail in ***FIG. 4*** below. Following the initial evaluation of the trait list using a control population, a second level evaluation is conducted using a population with well-characterized phenotypic differences ***205***. Data obtained from these plants is compared with the control data in order to determine if the observable differences in phenotype have been represented in the quantitative description ***206***. If the result of this comparison is not satisfactory then additional traits or characters may need to be defined and evaluated as above. If the results of the comparison are satisfactory then the traits analyzed meet the experimental criteria and are suitable for development as a high throughput platform ***207***.

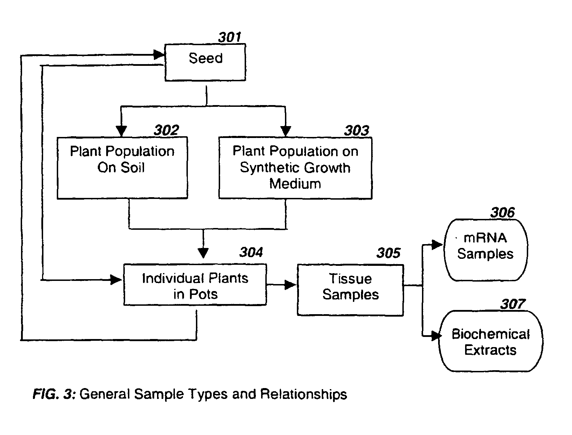

Development of a high throughput phenotypic analysis platform is dependent on a computerized sample tracking, work scheduling and data collection system. Each of these functions is accomplished by the LIMS system. Plant and plant-derived sample tracking is accomplished through a unique barcode that identifies each sample. Within the LIMS these barcodes are linked to database tables that contain information regarding the nature of the sample as well as its location and experimental history. Examples of sample types tracked by the LIMS are given in ***FIG. 3.*** Seeds ***301*** are the core sample type from which all plants and plant-derived samples originate. Depending on the configuration and design of the experiment, seeds may be sown to generate populations of plants growing on soil ***302*** or on synthetic defined growth media ***303***. After a period of growth and analysis, individual plants from these populations are selected for transplantation to single pots and tracked separately ***304***. As an alternative to transplantation, single seeds may be sown directly in individual pots ***304.*** In either case the individual plants are subjected to detailed phenotypic analysis as outlined in ***FIG***. ***4*** below. As part of this analysis plant tissue samples ***305*** are generated which are processed to yield RNA samples ***306*** and biochemical extracts ***307*** which are used for gene expression profiling and biochemical profiling, respectively. The LIMS table structure links each sample barcode to information detailing sample location and experimental status, as well as growth, treatment or storage conditions and genetic makeup and lineage. For instance if a plant is treated with a chemical for experimental purposes, this information is entered as part of the plant's history and is also included in the description of tissue samples derived from the treated plant. The LIMS also maintains a running log of the actual environmental conditions during the course of each experiment. This log includes actual temperature and humidity readings as well as data regarding adherence to watering, fertilization and light bulb rotation schedules.

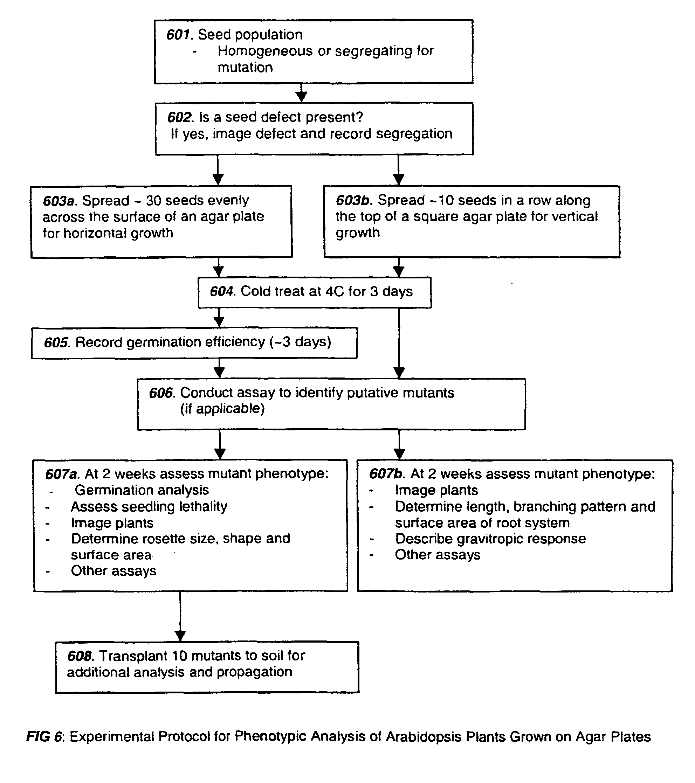

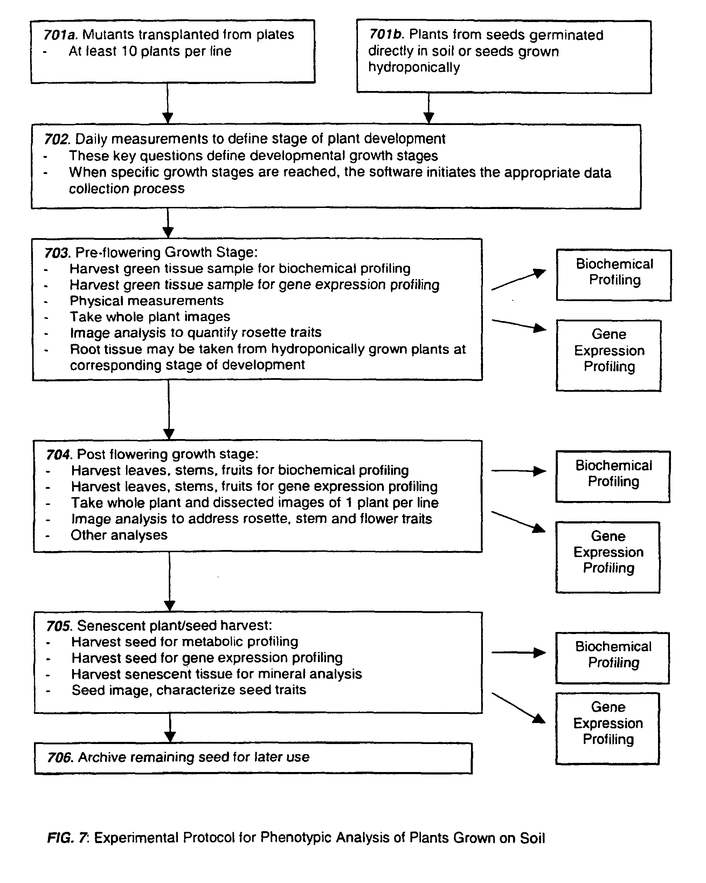

Workflow scheduling is based on specific experimental criteria. A general example of the logic involved in the process in given in **FIG. 4.** Experiments are typically initiated by sowing seeds or transplanting seedlings ***401***. The plants are grown under tightly controlled environmental conditions and at frequent intervals (*e.g*., daily) they are subjected to a core set of measurements ***402*** that can be performed rapidly, facilitating a throughput of hundreds of samples per day. These core measurements are derived from the larger data set described in ***FIG. 2*** above and are selected as being useful in defining specific growth stages. Data from the core measurements is compared to control data ***403*** in order to determine the developmental growth stage of the experimental plant. Prior to the onset of the experiment one or more growth stages are defined as being of interest for detailed comparative study. In a typical experimental protocol 3 to 5 growth stages may be chosen for detailed analysis. When plants of a test population reach one of these pre-defined growth stages ***404***, they are subjected to a detailed phenotypic analysis consisting of non-destructive measurements ***405.*** These measurements include physical measurements and image-based analyses, examples of which are detailed below in ***FIGS. 6 & 7.*** Additionally, a sub-population of the test population is harvested for destructive analysis ***406,*** including mass determination, gene expression profiling and biochemical profiling. Analysis of the entire plant life cycle is dependent on each experimental population containing sufficient individuals to allow destructive sampling at each of the pre-defined growth stages. A suggested listing of principal growth stages for plants together with a corresponding numerical designation in parentheses, includes germination (O), leaf production (1), cotyledon growth (3'), rosette growth (3), inflorescence emergence (5), flowering (6), fruit development (7), fruit ripening (8) and senescence (9).

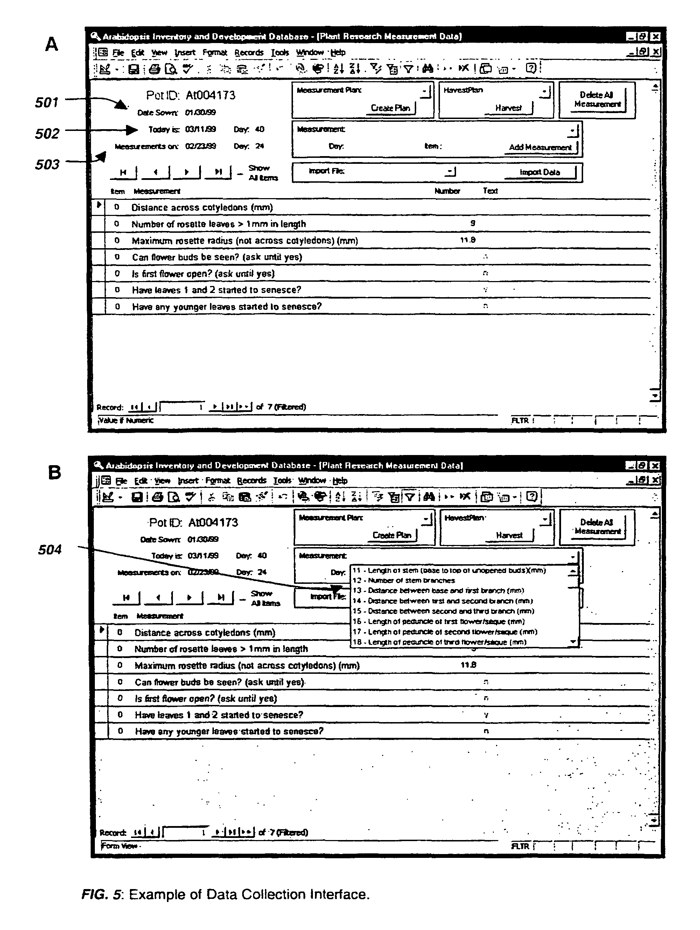

The LIMS provides a data collection interface that is derived from the workflow schedule and informs the user which measurements require completion for each sample and where appropriate, provides a corresponding field for data entry. A prototype example of a typical data collection interface is given in **FIG. 5A & B.** The interface depicts the day the plant was sown ***501,*** the current day ***502*** and the day for which the measurements shown were scheduled ***503.*** If necessary the measurement list can be modified by deleting measurements from the list, or by adding new measurements selected from the master measurement list ***504.*** Tasks for each workstation are provided independently, adding to the modular nature of the system.

The following section provides a detailed example of a phenotypic data collection platform designed to characterize mutant *Arabidopsis* plants over the entire life cycle of the plant. Each plant line enters the process as a population of seeds. This seed population may be homozygous or segregating for wild type and putative mutant phenotypes. The seeds are sown *in vitro* on agar plates and allowed to develop without selection. Morphological data reflecting the early developmental stages of these plants is collected at defined intervals. At ~2 weeks of age a subset of these plants is transplanted to soil for further developmental analysis. These plants are monitored closely, and at specific developmental time points detailed measurements are performed. Also at these time points, individual plants from these populations are harvested and the resulting tissue samples are sent for molecular phenotypic analyses (*e.g*., gene expression profiling, biochemical profiling, etc.).

An experimental protocol for the phenotypic characterization of *Arabidopsis* seedlings grown on plates is given in ***FIG. 6.*** Samples begin as seed populations ***601.*** The seed populations may be homogeneous, producing a population of genetically identical plants, or alternatively, they may be heterogeneous, producing a mixture of wild type and mutant seeds. At the time the seed is collected it must be examined for the presence of segregating abnormalities ***602.*** This inspection is done under the dissecting microscope and, if an obvious defect is noted, requires an image of the defective seeds to be captured for later analysis. The ratio of normal to abnormal seeds is entered into the LIMS as numeric data. Seeds are surface sterilized and plated on synthetic complete growth media. Seed from each line is analyzed in two different plate configurations to allow different aspects of development to be addressed. In the first configuration ***603a,*** ~30 seeds are positioned in a grid pattern on the surface of an agar plate. These plates are used to address development of the green part of the plant and also serve as a source of plants for transplantation to soil (see below). In the second configuration ***603b***, ~10 seeds are spaced evenly along the top edge of a square agar plate. This plate is placed at a 90-degree angle relative to gravity such that the roots grow along the surface of the agar. This growth configuration is used to address root growth and development. The plated seeds are cold treated for 3 days to foster germination consistency ***604.*** On the day wild type germination is typically complete (∼ day 5), the percentage of germination on the horizontal experimental plates is determined ***605***. This can be done manually or through image analysis. Poor germination may indicate the presence of an embryo lethal defect. If applicable, at ~ 2 weeks of age the plants are subjected to an assay to ascertain which plants are likely to be mutants ***606***. This assay is typically based on the detection reporter gene expression or activity that is indicative of the presence of a mutagenic transgene. Examples of reporter gene assays that may be used in this capacity are green fluorescence protein (GFP), herbicide or antibiotic resistance genes and morphological markers such as the presence or absence of leaf hairs (trichomes). Putative mutants are subjected to a more detailed developmental analysis at ~ 2 weeks of age. A primary reason for conducting developmental analysis on plates is the advantage it provides in assessing embryo and seedling lethal phenotypes. Therefore, particular attention must be given to seeds that fail to germinate or those that produce seedlings that die at an early age. In all cases, an image of at least 2 plants from each plate is captured for analysis. The analysis of plants grown on horizontal plates ***607a*** results in the following data for each plant: size of rosette, shape of rosette, total exposed leaf area and percent lesioned leaf area. Other assays may also be performed on these plates at this step including number of rosette leaves, and color quantitation. Additionally, fluorescence-based assays may be used to obtain a relative measure of photosynthetic capacity and in some cases may also be used to determine the relative abundance of certain fluorescent metabolites within the plants. Analysis of the plants grown on vertical plates ***607b*** results in the following data for each plant: branching pattern, surface area, and length of the root system, as well as a description of the gravitropic response of root system (vector of growth relative to gravity). Additional image analysis may be conducted to assess traits including root diameter and color. A minimum of 10 plants is transplanted from the horizontal plate to soil for additional analysis ***608***. The above process describes data collection from plants grown under standard conditions on complete synthetic medium. A similar set of analyses can be performed on plants grown on media designed to produce nutritional deficiencies or environmental stress.

A standard protocol for the analysis of Arabidopsis plants grown in soil in presented in ***FIG. 7.*** Typically, plant samples for this process come from the analysis of plants grown on agar plates ***701a*** as described in the preceding section. However, a subset of plant samples for inclusion in this process come from seeds that are germinated directly on soil or from seeds germinated and grown hydroponically ***701b***. As described in ***FIG***. ***4,*** each day core sets of traits are analyzed to define the growth stage of the experimental plants ***702.*** The resulting data is used by the LIMS to determine when specific growth stages are reached. In this example the first growth stage for detailed analysis is a pre-flowering growth stage defined by rosette leaf number ***703.*** When this stage is reached the LIMS schedules a series of detailed analyses, including the harvest of a sub-population to generate tissue samples for gene expression profiling biochemical profiling. From soil grown plants these samples are typically leaf tissue. Plants grown hydroponically are used to provide root tissue samples. Other plants are selected for non-destructive measurements, including physical and image-based analysis. These analyses include a determination of rosette size, shape and exposed surface area. The second growth stage of interest is a post-flowering stage that is determined primarily by flowering time ***704***. As in the pre-flowering stage analysis, a sub-population from each experimental population is harvested and tissue samples collected for gene expression and metabolic profile analysis. Tissue sample types analyzed at this point include leaves, stems and flowers or fruits. The plants selected previously for physical measurements and image analysis are analyzed again using the same techniques. Data is collected describing rosette size, shape, area and color. Additional data is collected regarding stem length and branching pattern, number and spacing of flowers/fruits, and floral structure. The final growth stage for analysis is the senescent stage at which the seeds are completely developed, desiccated and ready for harvest ***705***. Seed is harvested from each plant individually, weighed and counted. An image of the seeds is taken and analyzed to determine average seed size and shape. Seed samples are also submitted for gene expression and biochemical profiling analysis. Senescent tissue is harvested and analyzed for mineral content. The remaining seed is desiccated and stored for later use ***706.***

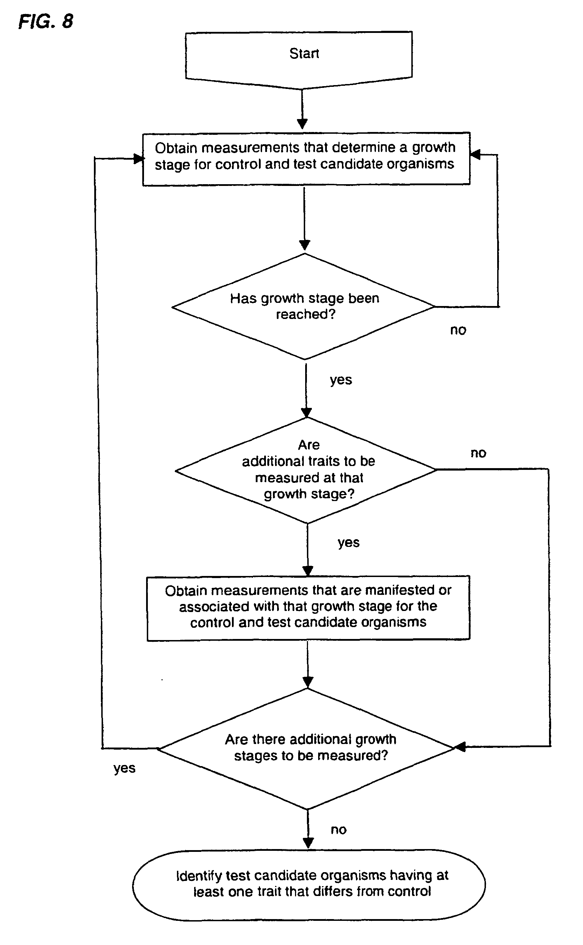

In **FIG. 8** a generic flow chart depicts a method for identifying a test candidate organism that has at least one trait that differs from a control population. In the first step, measurements are obtained for traits that determine a growth stage for a control population and for test candidate organisms. The organism can be a plant, insect, nematode, fungi, mouse or any other type of organism that is amenable to testing and analysis by the present method. If the organism is a plant, the plant can be either a monocot or a dicot plant. For a plant, the growth stage can be one or more of the following: germination, leaf production, cotyledon growth, rosette growth, inflorescence emergence, flowering, fruit development, fruit ripening, senescence or combinations thereof. In the second step, after measurements have been obtained, a determination is made regarding whether a growth stage has been reached, i.e. what stage of growth an organism is at based upon the measurements obtained. If yes, a particular growth stage has been reached, then a third step is initiated to determine what additional traits of the organism are to be measured at that growth stage. If no, a particular growth stage has not been reached, then the measurements that determine a growth stage for control and test organisms as described in the first step are repeated. If there are no additional traits to be measured at that growth stage and there are additional growth stages to be determined, then new measurements are taken for additional growth stage determination. In the first and the third steps, the traits of the organism that are to be measured can be developmental, morphological, visual, physiological, molecular, biochemical in nature or combinations thereof. A fourth step is also initiated to obtain measurements for traits as determined in the third step that are manifested or associated with that growth stage for the control and the test candidate organisms. For the first and fourth steps, measurements of traits can be collected using macroscopic photography, physical measurements, dissection, color quantification, microscopic photography, weight determination, image analysis, fluorescence spectroscopy measurements or combinations thereof. An optional and preferred fifth step is to determine whether there are any additional growth stages to be measured. If yes, then the first, second, third, and fourth steps are repeated for additional growth stages to be measured. When there are no additional growth stages to be measured, then the final step is to identify those test candidate organisms having at least one trait that differs from the control population based upon the measured traits of growth stage and/or traits that may be manifested or associated with at least one growth stage.

Although **FIG. 8** is presented to depict a general method for identifying a test candidate organism that has at least one trait that differs from a control population, the flow chart can be readily adapted to describe programmable methods, methods in computer systems, computer-readable media, computer systems, and computer memory which can be utilized to implement the general method. For example, in one embodiment, the present invention is directed to a method in a computer system for identifying a test candidate organism that has at least one trait that differs from a control population. In another embodiment, the present invention is directed to computer-readable medium containing instructions that enable the identification of a test candidate organism. In another embodiment, the present invention is directed to a computer-readable medium whose contents transform a computer system into a system for identifying a test candidate organism that has at least one trait that differs from a control population. In another embodiment, the present invention is directed toward a computer system programmed to identify a test candidate organism that has at least one trait that differs from a control population. In yet another embodiment, the present invention is directed toward a computer memory containing a data structure for storing data relating to traits of control and test candidate organisms. In the preferred embodiment of such computerized methods or systems, the computer drives the process by which measurements are collected or taken. That is, the computer and/or software determines what stage of growth an organism is at and what additional traits of the organism are to be measured at that growth stage, based upon measurements supplied to it by researchers. Alternatively, the process can be entirely manually driven, although the method would likely be carried out more slowly than if a computer were utilized. Optionally, the computer and/or software may be used to identify those test candidate organisms having at least one trait that differs from the control population. However, such identification can also be performed by individuals conducting the research.

Conventional analyses of statistical significance can be used to identify those test candidate organisms having at least one trait that differs from the control population based upon said measured traits of growth stage and/or traits that may be manifested or associated with at least one growth stage. Various types of statistical analyses are discussed in R. Steel and J. Torrie, Principles and Procedures of Statistics With Special Reference to the Biological Sciences, McGraw Hill Book Company, Inc. New York, (1960), 481 pages, including statistical analyses such as the standard deviation, t-tests, multiple range tests, multivariate analyses, linear regression, correlation and the like. For example, using the standard deviation for analysis, a trait of a test candidate organism could be said to differ from the control population if the trait of the test candidate organism falls outside about one standard deviation of a mean for the control population (also called the standard error of a mean). One of ordinary skill in the art can select those statistical analyses suitable for the experimental design.

We have emphasized the application of this method to the collection of morphological data. However, the same approach is applicable to the collection of tissue for subsequent molecular or biochemical analysis. It is likely that the regulation of the expression of nearly every gene and biochemical pathway is under some level of developmental control. Therefore, tissue harvests destined to supply material for metabolic or gene expression profiling will be much more useful if collected from different plant lines at similar developmental stages rather than from plants of similar chronological age. A representation of the developmental aspect is required for a more complete understanding of gene function.

The integration of data representing traits over the entire life cycle of plant into a single data set provides the opportunity to perform multivariate analysis and positions the investigator to identify correlative relationships among highly diverse traits at different stages of growth. Thus, it may be possible to identify easily measured and highly reproducible traits that can serve as surrogates for more difficult to measure or more variable traits, such as yield. Ultimately, these surrogate traits may be tested for validity in crop species and developed as transgenic products or as tools to aid in conventional breeding programs.

The data collection process presented here is amenable to high throughput implementation. The limited amount of effort required for first phase data collection facilitates the rapid determination of growth stages. Focusing on a subset of growth stages for the second phase data collection reduces the overall effort, while maintaining the ability to detect alterations in many different traits. Staggered sowings can be used to prevent all of the plants in a study from reaching growth stages for detailed analysis simultaneously, thereby allowing the analysis of many samples in parallel. In addition, the standardized rule sets employed in growth stage determination can readily be modeled in a computer application, enabling the creation of an interface that can be used to streamline the data collection process.

The following example illustrates the present invention in a manner in which it can be practised, but should not be construed as limiting the scope of the same.

### Example of the Invention

**Plant Growth**. All mutant lines are obtained from the Arabidopsis Resource Center (Columbus, Ohio). All plants are grown in Metro-Mix 200 (Scott's Sierra Horticultural Products) in individual 2.5 inch pots. Pots are arrayed in a 4X8 grid in standard greenhouse flats. Flats are grown on wire racks, each consisting of three 2x4-foot shelves. Each shelf provides space for 4 flats that are illuminated by 4-foot fluorescent tubes (SP61, General Electric Corp.) The day length is 16 hours and the average light intensity at the top of the pot is approximately 175 µmoles m⁻² s⁻¹. Day- and night-time temperatures are maintained at 22 and 20C, respectively. Relative humidity is maintained at 60-70%. Plants are watered by sub-irrigation as needed. Digital image analysis to obtain area, perimeter, S.D. radius, major axis, minor axis and eccentricity is performed using IP Lab Software (Scanalytics, Inc.).

**Growth stages.** Table 1 lists the growth stages that we have adapted from the BBCH scale for use in the analysis of *Arabidopsis* phenotypes. Together, these growth stages cover the development of the plant from the two-leaf stage through the completion of flowering and maturation of the first silique. Table 1 also shows the time required for wild-type Col-0 plants to reach each stage when grown under standard environmental conditions using a 16 h day length.

**Table 1.**

| **Growth stages for Arabidopsis** | | | | |
|---|---|---|---|---|
| | | **Col-0 Data** | | |
| **Stage** | **Description** | **Days** | **St. Dev.** | **CV** |
| Principal Growth Stage 1 | Leaf development | | | |
| 1.02 | 2 rosette leaves > 1 mm in length | 12.48 | 1.33 | 10.7 |
| 1.03 | 3 rosette leaves > 1 mm in length | 15.93 | 1.52 | 9.5 |
| 1.04 | 4 rosette leaves > 1 mm in length | 16.52 | 1.62 | 9.8 |
| 1.05 | 5 rosette leaves > 1 mm in length | 17.72 | 1.80 | 10.2 |
| 1.06 | 6 rosette leaves > 1 mm in length | 18.44 | 1.81 | 9.8 |
| 1.07 | 7 rosette leaves > 1 mm in length | 19.37 | 2.15 | 11.1 |
| 1.08 | 8 rosette leaves > 1 mm in length | 19.96 | 2.23 | 11.2 |
| 1.09 | 9 rosette leaves > 1 mm in length | 21.12 | 2.29 | 10.8 |
| 1.10 | 10 rosette leaves > 1 mm in length | 21.56 | 2.34 | 10.9 |
| 1.11 | 11 rosette leaves > 1 mm in length | 22.23 | 2.50 | 11.2 |
| 1.12 | 12 rosette leaves > 1 mm in length | 23.30 | 2.64 | 11.3 |
| 1.13 | 13 rosette leaves > 1 mm in length | 24.80 | 3.17 | 12.8 |
| 1.14 | 14 rosette leaves > 1 mm in length | 25.53 | 2.60 | 10.2 |
| Principal Growth Stage 3 | Rosette growth | | | |
| 3.20 | Rosette is 20% of final size | 18.88 | 3.02 | 16.0 |
| 3.50 | Rosette is 50% of final size | 23.96 | 4.07 | 17.0 |
| 3.70 | Rosette is 70% of final size | 27.36 | 4.10 | 15.0 |
| 3.90 | Rosette growth complete | 29.34 | 3.51 | 12.0 |
| Principal Growth Stage 5 | Inflorescence emergence | | | |
| 5.10 | First flower buds visible | 26.01 | 3.45 | 13.3 |
| Principal Growth Stage 6 | Flower production | | | |
| 6.00 | First flower open | 31.77 | 3.57 | 13.3 |
| 6.10 | 10% of flowers to be produced have opened | 35.86 | 4.86 | 13.6 |
| 6.30 | 30% of flowers to be produced have opened | 40.11 | 4.93 | 12.3 |
| 6.50 | 50% of flowers to be produced have opened | 43.52 | 4.87 | 11.2 |
| 6.90 | Flowering complete | 49.40 | 5.79 | 11.7 |
| Principal Growth Stage 8 | Silique Ripening | | | |
| 8.00 | First silique shattered | 47.98 | 4.47 | 9.3 |

Data from these measurements are collected every 48 hours during the life of the plant and are used in growth stage determination. These core measurements can be performed rapidly and require either a mechanical measurement with a caliper or ruler (*e.g*., maximum rosette radius) or a simple visual inspection (*e.g.*, number of rosette leaves).

Fig. 1A depicts growth stages and representative data from wild-type Col-0 plants. Schematic representation of chronological progression of principle growth stages. Horizontal bars indicate the period during wild-type Col-0 development when the indicated trait can be used in growth stage determination. Numbers in parentheses correspond to principle growth stages given in Table 1. As shown in Figure 1A, the defined growth stages are distributed evenly over the developmental timeframe, thereby maximizing the ability to detect subtle changes that affect only a limited aspect of development. Furthermore, the coefficients of variation (CV) associated with these data are generally less than 15% (Table 1), indicating the developmental progression of wild-type plants is highly reproducible. Together, these findings suggest this data set is a robust representation of wild-type development to which all mutants and environmentally stressed plants may be compared. Fig. 1B depicts the count or number of rosette leaves greater than 1 mm in length produced over time. Fig. 1C depicts the maximum rosette radius (*i.e.*, length of longest rosette leaf) over time. Fig. 1D depicts the stem height over time. Arrows indicate the time at which growth stages 6.00 and 6.50 occur. Data for (Figs. 1B-1D) are collected with a periodicity of two days and are representative of at least 300 individual plants. Days are given relative to date of sowing, including a three-day treatment at 4 degrees Celsius to synchronize seed germination.

Representative data taken from the analysis of wild-type Col-0 plants is given in Figure 1A. In most cases, growth stages can be assigned at the time of data acquisition. An example of this class is principal growth stage 1, in which the number of rosette leaves is the growth-stage-determining trait (Table 1 & Figure 1B). Traits such as this allow a clear determination of the growth stage at the time it is reached and serve as developmental landmarks that can be used to trigger second phase data collection activities. In contrast, other growth stage assignments are relative and can be determined unequivocally only in retrospect, after the relevant trait has developed to completion. Examples of this class are the principal stages 3 (Table 1; Fig. 1C) and 6 (Table 1). Growth stages of this type are defined as a percentage of the final value of the trait being measured. For example, growth stage 6.50 is reached when 50% of the final number of flowers have been produced. Without prior knowledge of the number of flowers that will be produced by a given plant, growth stage 6.50 can not be recognized at the time it occurs. Growth stage definitions of this category present a challenge if they are to be used in real time to trigger additional data collection activities, particularly if the plant under study is being characterized for the first time. In the case of stage 6.50 we have been able to develop an alternate definition that approximates the mid-flowering stage and allows it to be recognized at the time it occurs. As depicted by the arrow in the graph of stem length over time (Figure 1D), the first 50% of flower production in wild type Col-0 plants is completed coincident with a decrease in the rate of stem elongation. Extrapolating from this finding, we have established a working definition of stage 6.50 as the time at which the increase in stem elongation is less than 20% for 2 consecutive 48 hr data collection cycles. This working definition allows stage 6.50 to be used as a real-time trigger for the activation of additional data collection activities.

**Data collection model.** Our data collection model consists of two overlapping phases. In the first phase, a set of core measurements is performed at routine intervals over the course of development. These are measurements of traits that determine a growth stage of the control population and the test candidate organism, in this case, a plant. The resulting data reflect the rate of plant growth and development and are used in growth stage assignment. The second phase of the data collection process is triggered periodically throughout development as landmark growth stages are reached. These are measurements of traits of the organism that are manifested or associated with a particular growth stage for the control and the test candidate plant. Data collected during this phase reflect a wide range of morphological traits at several pre- and post-flowering growth stages. It is clear that not every phenotype can be represented adequately using the quantitative measurements presented here (Tables 2 & 3). As an example, qualitative characters such as altered leaf phyllotaxy or abnormal stem thickenings will not be represented directly in the quantitative data set. However, these data can be captured in images that are tagged with key word descriptors and stored for later reference.

**Growth Stage Measurements.** The first phase measurements are listed in Table 2.

**Table 2.**

| **First phase measurements** | |
|---|---|
| Growth stage defined | Measurement/ Query |
| Principal growth stage 1 | Number of rosette leaves > 1 mm |
| Principal growth stage 3 | Rosette radius |
| Stage 5.10 | Are flower buds visible? |
| Stage 6.00 | Is first flower open? |
| Stage 6.50^{a} | Length of stem |
| Principal growth stage 6 | Number of open flowers |
| Principal growth stage 6 | Number of senescent flowers |
| Principal growth stages 6 and 7 | Number of filled siliques |
| Principal growth stage 8 | Number of shattered siliques |
| Stage 6.90 | Is flower production complete? |

| | |
|---|---|
| ^{a} Used to define the working definition of stage 6.50 as described in the text | |

Table 3 lists the second phase data collection steps triggered at each of these growth stages and provides representative data from the analysis of wild type Col-0 plants.

**Table 3.**

| **Second phase measurements.** | | | | | |
|---|---|---|---|---|---|
| **Growth Stage** | **Measurement** | **Unit** | **Col-0 Data** | | |
| | | | **Average** | **St. Dev.** | **CV** |
| 1.04 | Number of cotyledons | count | 2.0 | 0.1 | 5.0 |
| 1.10 | Rosette - total exposed leaf area | mm² | 580.0 | 202.2 | 34.9 |
| 1.10 | Rosette - perimeter | mm | 418.0 | 119.1 | 28.5 |
| 1.10 | Rosette - s.d. radius | n.a. | 45.7 | 3.2 | 7.0 |
| 1.10 | Rosette - major axis | mm | 40.4 | 8.0 | 19.8 |
| 1.10 | Rosette - minor axis | mm | 34.6 | 7.5 | 21.7 |
| 1.10 | Rosette - eccentricity | n.a. | 0.5 | 0.1 | 20.0 |
| 6.00 | Rosette - total exposed leaf area | mm² | 3225.0 | 1088.3 | 33.7 |
| 6.00 | Rosette - perimeter | mm | 808.1 | 181.3 | 22.4 |
| 6.00 | Rosette - s.d. radius | n.a. | 36.7 | 3.5 | 9.5 |
| 6.00 | Rosette - major axis | mm | 82.3 | 15.3 | 18.6 |
| 6.00 | Rosette - minor axis | mm | 73.1 | 13.4 | 18.3 |
| 6.00 | Rosette - eccentricity | n.a. | 0.4 | 0.1 | 25.0 |
| 6.00 | Rosette - dry weight | mg | 117.4 | 45.9 | 39.1 |
| 6.50 | Number of stem branches on main bolt | count | 3.4 | 0.6 | 17.6 |
| 6.50 | Number of side bolts > 1 cm | count | 4.2 | 1.2 | 28.6 |
| 6.50 | Length of peduncle of second flower on main bolt | mm | 11.5 | 1.6 | 13.9 |
| 6.50 | Distance across face of open flower | mm | 3.9 | 0.3 | 7.7 |
| 6.50 | Sepal length | mm | 2.2 | 0.2 | 9.1 |
| 6.50 | Pollen grain - area | •m² | 589.0 | 132.0 | 22.4 |
| 6.50 | Pollen grain - perimeter | n.a. | 114.5 | 13.0 | 11.2 |
| 6.50 | Pollen grain - s.d. radius | •m | 9.1 | 2.5 | 27.5 |
| 6.50 | Pollen grain - major axis | •m | 30.6 | 3.3 | 10.8 |
| 6.50 | Pollen grain - minor axis | •m | 24.3 | 3.0 | 12.3 |
| 6.50 | Pollen grain - eccentricity | n.a. | 0.6 | 0.1 | 16.7 |
| 6.50 | Silique - area | mm² | 10.6 | 1.9 | 17.9 |
| 6.50 | Silique - perimeter | mm | 40.9 | 6.1 | 14.9 |
| 6.50 | Silique - s.d. radius | n.a. | 55.8 | 0.5 | 0.9 |
| 6.50 | Silique - major axis | mm | 17.2 | 1.7 | 9.9 |
| 6.50 | Silique - minor axis | mm | 1.2 | 0.2 | 16.7 |
| 6.50 | Silique - eccentricity | n.a. | 1.0 | 0.0 | 0.0 |
| 6.50 | Total number of seeds per silique valve | count | 29.9 | 2.8 | 9.4 |
| 6.50 | Number of abnormal seeds per silique valve | count | 0.2 | 0.4 | 200 |
| 6.50 | Dry weight of stem | mg | 188.8 | 39.3 | 20.8 |
| 6.50 | Dry weight of rosette | mg | 163.7 | 52.0 | 31.8 |
| 6.90 | Total number of siliques | Count | 160.4 | 60.7 | 37.8 |
| 9.70 | Seed -area | mm² | 0.14 | 0.01 | 7.1 |
| 9.70 | Seed - perimeter | mm | 1.95 | 0.04 | 2.1 |
| 9.70 | Seed - s.d. radius | n.a. | 16.92 | 0.94 | 5.6 |
| 9.70 | Seed - major axis | mm | 0.53 | 0.03 | 5.7 |
| 9.70 | Seed - minor axis | mm | 0.33 | 0.02 | 6.1 |
| 9.70 | Seed - eccentricity | n.a. | 0.78 | 0.02 | 2.6 |
| 9.70 | Seed yield per plant (desiccated) | mg | 127.9 | 52.7 | 41.2 |

Of the forty-three traits listed in Table 3, fifteen have CVs that are less than 10%. Traits in this category include flower size, number of seeds per silique and seed size and shape characters. Twenty-one traits have CVs that are between 10 and 30%. These include size and shape estimates for rosettes and pollen grains, inflorescence structure characters (*e.g*., number of stem branches), as well as silique area and perimeter. Of the traits listed in Table 3, only seven exhibit CVs above 30%. Notably, these include exposed rosette leaf area and dry weight, total number of siliques and mass of seeds produced (yield). Thus, the total number of siliques produced by the plant (CV = 37.8%) plays a greater role in the variation associated with yield than does the number of seeds per silique (CV = 9.4%). Consistent with this finding, we have been unable to reduce the variation associated with yield through modified harvesting procedures designed to account for seed loss as a result of pre-mature silique dehiscence.

**Measurements for traits of the organism that are manifested or associated with a growth stage for control and test candidate organisms**. The second phase of the data collection process is designed to identify phenotypes in traits other than those utilized in growth stage determination. The growth stages 1.04, 1.10, 6.00, 6.50, 6.90 and 9.70 are chosen as triggers for second phase data collection. These stages represent key developmental points in the *Arabidopsis* life cycle and can be detected readily at the time they occur. Stages 1.04 and 1.10 represent early and late pre-flowering stages of growth, respectively, while stages 6.00, 6.50 and 6.90 span the period of flower production. Traits related to seed yield and quality are assessed at stage 9.70.

**Validation of the method**. We addressed our ability to quantify phenotypic differences by analyzing the developmental progression of five mutant lines that were obtained from the Arabidopsis Biological Resource Center (Columbus, Ohio). The time required for each mutant to reach growth stages 1.04, 1.10, 5.10, 6.00 and 6.90 was determined and is presented graphically in Figure 2A, which provides a summary of growth stage progression for wild type (Col-0) and five mutant lines. Arrows define the time at which Col-0 plants reached the growth stages indicated. Boxes represent the time elapsed between the occurrence of successive growth stages. Junctions between boxes of different patterns indicate the occurrence of a growth stage.

A comparison of the developmental time lines of these mutants reveals striking differences between the mutants and wild type Col-0. The *hls1-1* mutant has reduced ethylene production and lacks a pronounced apical hook when grown in the dark (Guzman and Ecker, 1990). Our data from the analysis of light-grown *hls1-1* plants are in agreement with previous reports (ref) in that they reveal an early flowering phenotype when compared to the Col-0 control (stages 5.10 & 6.00; Fig. 2A). In spite of this early flowering phenotype, *hls1-1* plants flower for a longer period of time, such that both Col-0 and *hls1-1* cease flower production (stage 6.90) at approximately 49 days after sowing. The *fae1-1* mutant was identified as having a reduced content of very-long-chain-fatty-acids in the seed (Lemieux et. al., 1990). No vegetative phenotypes have been ascribed to the *fae1-1* mutation and biochemical and mRNA expression studies have suggested that the activity of the *FAE1* gene is restricted to the seed (James, D.W., Lim, E., Keller, J., Plooy, I., Ralston, E. & Dooner, H.K. Directed tagging of the *Arabidopsis FATTY ACID ELONGATION (FAE1)* gene with the maize transposon Activator. *Plant Cell* 7, 309-319 (1995)). Surprisingly, our analysis reveals that *fae1-1* plants make the transition to flowering (stage 5.10) sooner than Col-0. In addition, *fae1-1* reaches stage 5.10 prior to stage 1.10, suggesting that *fae1-1* plants produce fewer rosette leaves than Col-0. These data suggest that *fae1-1* or a tightly linked mutation may have a role in development. The *fah1-2* (Chapple, C.C.S., Vogt, T., Ellis, B.E. & Somerville, C.R. An *Arabidopsis* mutant defective in the general phenylpropanoid pathway. *Plant Cell* 4, 1413-1424 (1992)), *cgl1-1* (von Schaewen, A., Sturm, A., O'Neil, J. & Chrispeels, M.J. Isolation of a mutant Arabidopsis plant that lacks N-acetyl glucosaminyl transferase I and is unable to synthesize golgi-modified complex N-linked glycans. *Plant Physiol.* 102, 1109-1118 (1993)) and *adg1-1* (Lin, T.-P., Caspar, T., Somerville, C. & Preiss, J. Isolation and characterization of a starchless mutant of *Arabidopsis thaliana* (L.) Heynh lacking ADPglucose pyrophosphorylase activity. *Plant Physiol*. 86, 1131-1135 (1988)) mutants were also selected via biochemical screening procedures and have alterations in phenylpropanoid metabolism, complex glycan biosynthesis and starch content, respectively. No visible phenotypes have been reported for these mutants. However, our analysis revealed subtle changes in their developmental progression (Fig. 2A).

In Fig. 2B is shown a comparison of silique area and the number seeds per half silique between *adg1-1* and Col-0. Silique area was obtained *via* computerized analysis of digital images of mature filled siliques. The number of seeds per half silique was observed following removal of the outer layer of one valve of a mature filled silique. Both measures were averaged from three siliques per plant and are reported as the the average of 10 or >300 plants for *adg1-1* and Col-0, respectively.

In Fig. 2C is shown a comparison of silique number and the yield per plant between *adg1-1* and Col-0. The final number of siliques per plant was determined following the completion of flower production (stage 6.90). Yield is reported as the desiccated mass (mg) of seeds produced per plant. Data are averages and standard deviations of 10 or >300 plants for *adg1-1* or Col-0, respectively.

## Claims

1. A method for identifying a test candidate organism that has at least one trait that differs from a control population, the method comprising:
a) establishing the stages of growth for an individual control organism or for a population of control organisms and the criteria for determination thereof;
b) programming a computer to identify what stage of growth an individual organism is at or a population of organisms is at, based upon the criteria established in step a);
c) establishing the traits of an organism that may be manifested or associated with said growth stages and the criteria for measurement thereof;
d) programming a computer to identify what additional traits of an organism are to be measured according to the criteria established in step c);
e) growing control organisms and at least one test candidate organism;
f) providing measurements of growth stage determining traits from said control organisms and said test candidate organism to said computer that will enable the computer to specify
i) what stage of growth an organism is at; and
ii) what traits of the organism are to be measured at that growth stage;
g) measuring those traits specified by the computer for control and test candidate organisms;
h) providing data on the measurements of those traits to the computer;
i) optionally, repeating steps f), g) and h) for additional growth stages to be measured; and
j) identifying those test candidates plants having at least one trait that differs from the control population based upon said measured traits of growth stage and/or traits that may be manifested or associated with at least one growth stage.

2. A computerized method for identifying a test candidate organism that has at least one trait that differs from a control population, the method comprising:
a) obtaining measurements of traits that determine a growth stage for said control population and said test candidate organism;
b) optionally, determining what stage of growth an organism is at based upon measurements obtained in step a);
c) determining what additional traits of the organism are to be measured at that growth stage;
d) obtaining measurements for traits as determined in step c) that are manifested or associated with that growth stage for said control and said test candidate organisms;
e) optionally, repeating stages a), b), c) and d) for additional growth stages to be measured; and
f) optionally, identifying those test candidate organisms having at least one trait that differs from the control population based upon said measured traits of growth stage and/or traits that may be manifested or associated with at least one growth stage.

3. A computer-readable medium containing instructions for a method to enable the identification of a test candidate organism having at least one trait that differs from a control population, the method comprising:
a) obtaining measurements of traits that determine a growth stage for said control population and said test candidate organism;
b) optionally, determining what stage of growth an organism is at based upon measurements obtained in step a);
c) determining what additional traits of the organism are to be measured at that growth stage;
d) obtaining measurements for traits as determined in step c) that are manifested or associated with that growth stage for said control and said test candidate organisms;
e) optionally, repeating steps a), b), c) and d) for additional growth stages to be measured; and
f) optionally, identifying those test candidate organisms having at least one trait that differs from the control population based upon said measured traits of growth stage and/or traits that may be manifested or associated with at least one growth stage.

4. The computer-readable medium of Claim 3, wherein the organism is a plant.

5. The computer-readable medium of Claim 3, wherein the organism is a dicot or a monocot plant.

6. The computer-readable medium of Claim 3, wherein the organism is a dicot plant.

7. The computer-readable medium of Claim 3, wherein a growth stage is one or more of the following: germination, leaf production, cotyledon growth, rosette growth, infloresence emergence, flowering, fruit development, fruit ripening, senenscence or combinations thereof.

8. The computer-readable medium of Claim 3, wherein the traits of the organism that are to be measured are from one or more of the following characteristics: developmental, morphological, visual, physiological, molecular, biochemical in nature or combinations thereof.

9. The method of Claim 2, wherein measurements of traits are collected using one or more of the following: macroscopic photography, physical measurements, dissection, color quantification, microscopic photography, weight determination, image analysis, fluorescence spectroscopy measurements or combinations thereof.

10. A computer-readable medium whose contents transform a computer system into a system for identifying a test candidate organism that has at least one trait that differs from a control population, the medium comprising:
a) a subsystem which receives measurements of growth stage determining traits;
b) an optional subsystem which determines what stage of growth an organism is at based upon measurements received in subsystem a);
c) a subsystem which specifies what traits of the organism are to be measured at that growth stage;
d) a subsystem which receives measurements of traits of the organism to be measured at a growth stage for said control and said test candidate organisms;
e) an optional subsystem which can repeat steps a), b), c) and d) for additional growth stages to be measured; and
f) an optional subsystem which can identify those test candidate organisms having at least one trait that differs from the control population based upon said measured traits of growth stage and/or traits that may be manifested or associated with at least one growth stage.

11. A computer system programmed to identify a test candidate organism that has at least one trait that differs from a control population, the system comprising:
a) a subsystem which receives measurements of growth stage determining traits;
b) an optional subsystem which determines what stage of growth an organism is at based upon measurements received in subsystem a);
c) a subsystem which specifies what traits of the organism are to be measured at that growth stage;
d) a subsystem which receives measurements of traits of the organism to be measured at a growth stage for said control and said test candidate organisms;
e) an optional subsystem which can repeat steps a), b), c) and d) for additional growth stages to be measured; and
f) an optional subsystem which can identify those test candidate organisms having at least one trait that differs from the control population based upon said measured traits of growth stage and/or traits that may be manifested or associated with at least one growth stage.

12. A method for identifying a test candidate organism that has at least one trait that differs from a control population, the method comprising:
a) obtaining measurements of traits that determine a growth stage for said control population and said test candidate organism;
b) determining what stage of growth an organism is at based upon measurements obtained in step a);
c) determining what additional traits of the organism are to be measured at that growth stage;
d) obtaining measurements for traits as determined in step c) that are manifested or associated with that growth stage for said control and said test candidate organisms;
e) optionally, repeating steps a), b), c) and d) for additional growth stages to be measured; and
f) identifying those test candidate organisms having at least one trait that differs from the control population based upon said measured traits of growth stage and/or traits that may be manifested or associated with at least one growth stage.

13. The method of Claim 1, 2 or 12, wherein the organism is a plant.

14. The method of Claim 1, 2 or 12, wherein the organism is a dicot or a monocot plant.

15. The method of Claim 1, 2 or 12, wherein the organism is a dicot plant.

16. The method of Claim 15, wherein the growth stage is one or more of the following: germination, leaf production, cotyledon growth, rosette growth, infloresence emergence, flowering, fruit development, fruit ripening, senenscence, or combinations thereof.

17. The method of Claims 1, 2 or 12, wherein the traits of the organism that are to be measured are from one or more of the following characteristics: developmental, morphological, visual, physiological, molecular, biochemical in nature or combinations thereof.

18. The method of Claim 1, 2 or 12, wherein measurements of traits are collected using one or more of the following: macroscopic photography, physical measurements, dissection, color quantification, microscopic photography, weight determination, image analysis, fluorescence spectroscopy measurements or combinations thereof.

## Patentansprüche

1. Verfahren zum Identifizieren eines Prüflingsorganismus, der wenigstens eine Charakteristik aufweist, die von einer Kontrollprobe abweicht, wobei das Verfahren umfasst:
a) Festsetzen der Wachstumsstadien für einen individuellen Kontrollorganismus oder für einen Stichprobenumfang von Kontrollorganismen und die Kriterien für dessen bzw. deren Bestimmung;
b) Programmieren eines Computers, um, auf den in Schritt a) festgesetzten Kriterien beruhend, zu identifizieren, in welchem Wachstumsstadium ein individueller Organismus ist bzw. ein Stichprobenumfang des Organismus ist;
c) Festsetzen der Charakteristiken eines Organismus, die sich in besagten Wachstumsstadien zeigen können bzw. damit verbunden sind und der Kriterien zu deren Messung;
d) Programmieren eines Computers, um zu identifizieren, welche zusätzlichen Charakteristiken eines Organismus, gemäß den in Schritt c) festgesetzten Kriterien, gemessen werden sollen;
e) Wachstumskontrollorganismen und wenigstens einen Prüflingsorganismus;
f) Bereitstellen von Messungen des Wachstumsstadiums, die Charakteristiken seitens besagter Kontrollorganismen und besagtem Prüflingsorganismus bestimmen, an besagten Computer, die den Computer befähigen werden zu spezifizieren,
i) in welchem Wachstumsstadium sich ein Organismus befindet; und
ii) welche Charakteristiken des Organismus in welchem Wachstumsstadium gemessen werden sollen;
g) Messen jener Charakteristiken, die vom Computer für Kontroll- und Prüflingsorganismen spezifiziert wurden;
h) Bereitstellen von Daten über die Messungen jener Charakteristiken an den Computer;
i) wahlweise - Wiederholen der Schritte f), g) und h) für zu messende zusätzliche Wachstumsstadien; und
j) Identifizieren jener Prüflingspflanzen, die wenigstens eine Charakteristik aufweisen, die, beruhend auf besagten gemessenen Charakteristiken des Wachstumsstadiums und/oder der Charakteristiken, die sich in wenigstens einem Wachstumsstadium zeigen bzw. damit verbunden sind, von der Kontrollprobe abweicht

2. Computerisiertes Verfahren zum Identifizieren eines Prüflingsorganismus, der wenigstens eine Charakteristik hat, die von einer Kontrollprobe abweicht, wobei das Verfahren umfasst:
a) Beschaffen von Messungen der Charakteristiken, die ein Wachstumsstadium für besagte Kontrollprobe und besagten Prüflingsorganismus bestimmen;
b) wahlweise - Bestimmen in welchem Wachstumsstadium sich ein Organismus, beruhend auf den bei Schritt a) erhaltenen Messungen, befindet;
c) Bestimmen, welche zusätzlichen Charakteristiken des Organismus in jenem Wachstumsstadium gemessen werden sollen;
d) Beschaffen von Messungen für Charakteristiken wie bei Schritt c) bestimmt, die sich in jenem Wachstumsstadium für besagte Kontroll- und besagte Prüflingsorganismen zeigen bzw. damit verbunden sind;
e) wahlweise - Wiederholen der Schritte a), b), c) und d) für zu messende zusätzliche Wachstumsstadien; und
f) wahlweise - Identifizieren jener Prüflingsorganismen, die wenigstens eine Charakteristik aufweisen, die, beruhend auf besagten gemessenen Charakteristiken des Wachstumsstadiums und/oder der Charakteristiken, die sich in wenigstens einem Wachstumsstadium zeigen können bzw. damit verbunden sind, von der Kontrollprobe abweicht.

3. Computerlesbares Medium, das Instruktionen für ein Verfahren enthält, um die Identifizierung eines Prüflingsorganismus mit wenigstens einer Charakteristik zu befähigen, die von einer Kontrollprobe abweicht, wobei das Verfahren umfasst:
a) Beschaffen von Messungen der Charakteristiken, die ein Wachstumsstadium für besagte Kontrollprobe und besagten Prüflingsorganismus bestimmen;
b) wahlweise - Bestimmen in welchem Wachstumsstadium sich ein Organismus, beruhend auf den bei Schritt a) erhaltenen Messungen, befindet;
c) Bestimmen welche zusätzlichen Charakteristiken des Organismus in jenem Wachstumsstadium gemessen werden sollen;
d) Beschaffen von Messungen für Charakteristiken wie bei Schritt c) bestimmt, die sich in jenem Wachstumsstadium für besagte Kontroll- und besagte Prüflingsorganismen zeigen bzw. damit verbunden sind;
e) wahlweise - Wiederholen der Schritte a), b), c) und d) für zu messende zusätzliche Wachstumsstadien; und
f) wahlweise - Identifizieren jener Prüflingsorganismen, die wenigstens eine Charakteristik aufweisen, die, beruhend auf besagten gemessenen Charakteristiken des Wachstumsstadiums und/oder Charakteristiken, die sich in wenigstens einem Wachstumsstadium zeigen können bzw. damit verbunden sind, von der Kontrollprobe abweicht.

4. Das computerlesbare Medium des Anspruchs 3, wobei der Organismus eine Pflanze ist.

5. Das computerlesbare Medium des Anspruchs 3, wobei der Organismus eine zweikeimblättrige oder eine einkeimblättrige Pflanze ist.

6. Das computerlesbare Medium des Anspruchs 3, wobei der Organismus eine zweikeimblättrige Pflanze ist.

7. Das computerlesbare Medium des Anspruchs 3, wobei ein Wachstumsstadium eins oder mehr von Folgendem ist: Keimung, Blattproduktion, Keimblattwachstum, Rosettenwachstum, Infloreszenzaufgang, Blütezeit, Fruchtentwicklung, Fruchtreifung, Seneszenz (Alterung) oder Kombinationen davon.

8. Das computerlesbare Medium des Anspruchs 3, wobei die zu messenden Charakteristiken des Organismus eine oder mehrere der folgenden Charakteristiken sind: Entwicklung betreffend, morphologischer, visueller, physiologischer, molekularer, biochemischer Natur oder Kombinationen davon.

9. Das Verfahren des Anspruchs 2, wobei Messungen von Charakteristiken bzw. Merkmalen unter Verwendung von einem oder mehreren des Folgenden gesammelt werden: makroskopische Fotografie, physikalische Messungen, Präparation, Farbquantifizierung, mikroskopische Fotografie, Gewichtsbestimmung, Bildanalyse, Fluoreszenzspektroskopiemessungen oder Kombinationen davon.

10. Ein computerlesbares Medium, dessen Inhalt ein Computersystem in ein System für das Identifizieren eines Prüflingsorganismus verwandelt, der wenigstens eine Charakteristik hat, die sich von einer Kontrollprobe unterscheidet, wobei das Medium umfasst:
a) ein Subsystem, das Messungen von Wachstumsstadium bestimmenden Charakteristiken erhält;
b) ein wahlweises Subsystem, das bestimmt in welchem Wachstumsstadium sich ein Organismus, beruhend auf im Subsystem a) erhaltenen Messungen, befindet;
c) ein Subsystem, das spezifiziert welche Charakteristiken des Organismus in diesem Wachstumsstadium zu messen sind;
d) ein Subsystem, das Messungen von Charakteristiken des Organismus erhält, die in einem Wachstumsstadium für besagte Kontroll- und Prüflingsorganismen zu messen sind;
e) ein wahlweises Subsystem, das die Schritte a), b), c) und d) für zu messende zusätzliche Wachstumsstadien wiederholen kann; und
f) ein wahlweises Subsystem, das jene Prüflingsorganismen identifizieren kann, die wenigstens eine Charakteristik aufweisen, die, beruhend auf besagten gemessenen Charakteristiken des Wachstumsstadiums und/oder Charakteristiken, die sich in wenigstens einem Wachstumsstadium zeigen können bzw. damit verbunden sind, von der Kontrollprobe abweicht.

11. Ein Computersystem, das programmiert ist einen Prüflingsorganismus zu identifizieren, der wenigstens eine Charakteristik hat, die von einer Kontrollprobe abweicht, wobei das System umfasst:
a) ein Subsystem, das Messungen von Wachstumsstadium bestimmenden Charakteristiken erhält;
b) ein wahlweises Subsystem, das bestimmt in welchem Wachstumsstadium sich ein Organismus, beruhend auf im Subsystem a) erhaltenen Messungen, befindet;
c) ein Subsystem, das spezifiziert welche Charakteristiken des Organismus in diesem Wachstumsstadium zu messen sind;
d) ein Subsystem, das Messungen von Charakteristiken des Organismus erhält, die in einem Wachstumsstadium für besagte Kontroll- und Prüflingsorganismen zu messen sind;
e) ein wahlweises Subsystem, das die Schritte a), b), c) und d) für zu messende zusätzliche Wachstumsstadien wiederholen kann; und
f) ein wahlweises Subsystem, das jene Prüflingsorganismen identifizieren kann, die wenigstens eine Charakteristik aufweisen, die, beruhend auf besagten gemessenen Charakteristiken des Wachstumsstadiums und/oder Charakteristiken, die sich in wenigstens einem Wachstumsstadium zeigen können bzw. damit verbunden sind, von der Kontrollprobe abweicht.

12. Verfahren um einen Prüflingsorganismus zu identifizieren, der wenigstens eine Charakteristik aufweist, die von einer Kontrollprobe abweicht, wobei das Verfahren umfasst:
a) Beschaffen von Messungen der Charakteristiken, die ein Wachstumsstadium für besagte Kontrollprobe und besagten Prüflingsorganismus bestimmen; b) Bestimmen, in welchem Wachstumsstadium sich ein Organismus, beruhend auf Messungen befindet, die bei Schritt a) erhalten wurden;
c) Bestimmen welche zusätzlichen Charakteristiken des Organismus in jenem Wachstumsstadium gemessen werden sollen;
d) Beschaffen von Messungen für Charakteristiken wie bei Schritt c) bestimmt, die sich in jenem Wachstumsstadium für besagte Kontroll- und besagte Prüflingsorganismen zeigen bzw. damit verbunden sind;
e) wahlweise - Wiederholen der Schritte a), b), c) und d) für zu messende zusätzliche Wachstumsstadien; und
f) Identifizieren jener Prüflingsorganismen, die wenigstens eine Charakteristik aufweisen, die, beruhend auf besagten gemessenen Charakteristiken des Wachstumsstadiums und/oder Charakteristiken, die sich in wenigstens einem Wachstumsstadium zeigen können bzw. damit verbunden sind, von der Kontrollprobe abweicht.

13. Das Verfahren von Anspruch 1, 2 der 12, wobei der Organismus eine Pflanze ist.

14. Das Verfahren von Anspruch 1, 2 der 12, wobei der Organismus eine zweikeimblättrige oder eine einkeimblättrige Pflanze ist.

15. Das Verfahren von Anspruch 1, 2 der 12, wobei der Organismus eine zweikeimblättrige Pflanze ist.

16. Das Verfahren von Anspruch 15, wobei das Wachstumsstadium eins oder mehreren des Folgenden ist:
Keimung, Blattproduktion, Keimblattwachstum, Rosettenwachstum, Infloreszenzaufgang, Blütezeit, Fruchtentwicklung, Fruchtreifung, Seneszenz (Alterung) oder Kombinationen davon.

17. Das Verfahren der Ansprüche 1, 2 oder 12, wobei die zu messenden Charakteristiken des Organismus eine oder mehrere der folgenden Charakteristiken sind: Entwicklung betreffend, morphologischer, visueller, physiologischer, molekularer, biochemischer Natur oder Kombinationen davon.

18. Das Verfahren des Anspruchs 1, 2 oder 12, wobei Messungen von Charakteristiken bzw. Merkmalen unter Verwendung von einem oder mehreren des Folgenden gesammelt werden: makroskopische Fotografie, physikalische Messungen, Präparation, Farbquantifizierung, mikroskopische Fotografie, Gewichtsbestimmung, Bildanalyse, Fluoreszenzspektroskopiemessungen oder Kombinationen davon.

## Revendications

1. Une méthode pour identifier un organisme candidat de vérification qui a au moins un trait qui s'écarte d'une population de contrôle, la méthode comprenant:
a) établissant les étapes de croissance pour un organisme de contrôle individuel ou pour une population d'organismes de contrôle et les critères pour leur détermination;
b) programmant un ordinateur pour identifier à quelle étape de croissance un organisme individuel ou une population d'organismes se trouvent, sur la base des critères établis dans l'étape a);
c) établissant les traits d'un organisme qui peut être manifesté ou associé avec ces étapes de croissance, ainsi que les critères pour les mesurer;
d) programmant un ordinateur pour décider quels traits supplémentaires doivent être mesurés selon les critères établis dans l'étape c);
e) cultivant des organismes de contrôle et au moins un organisme candidat de vérification;
f) fournissant des mesures de traits déterminants d'étapes de croissance à partir de ces organismes de contrôle et de cet organisme de vérification à l'ordinateur, permettant l'ordinateur de spécifier
i) à quelle étape de croissance se trouve l'organisme; et
ii) quels traits de l'organisme doivent être mesurés à cette étape de croissance;
g) mesurant ces traits spécifiés par l'ordinateur pour les organismes de contrôle et de vérification;
h) fournissant des données sur les mesures de ces traits à l'ordinateur;
i) facultativement, répétant les étapes f), g) et h) pour mesurer des étapes de croissance supplémentaires; et
j) identifiant ces plantes candidates de vérification ayant au moins un trait qui s'écarte de la population de contrôle sur la base des traits d'étape de croissance mesurés et/ou de traits qui peuvent se manifester ou être associés à au moins une étape de croissance.

2. Une méthode assistée d'un ordinateur pour identifier un organisme candidat de vérification qui présente au moins un trait différent d'une population de contrôle, la méthode comprenant:
a) obtenant des mesures de traits qui déterminent une étape de croissance pour cette population de contrôle et cet organisme candidat de vérification;
b) facultativement, déterminant à quel stade de croissance est l'organisme, sur la base de mesures obtenues dans l'étape a);
c) déterminant quels traits supplémentaires de l'organisme doivent être mesurés à ce stage de croissance;
d) obtenant des mesures pour les traits détermines à l'étape c) qui se sont manifestés ou associés à ce stade de croissance pour les organismes de contrôle et de vérification;
e) facultativement, répétant les étapes a), b), c) et d) pour des stades de croissance supplémentaires à mesurer; et
f) facultativement, identifiant les organismes candidats de vérification ayant au moins un trait qui s'écarte de la population de contrôle basée sur ces traits mesurés de stade de croissance et/ou des traits pouvant se manifester ou être associés à au moins un stade de croissance.

3. Un milieu qu'un ordinateur peut lire contenant des instructions pour une méthode pour permettre l'identification d'un organisme candidat de vérification ayant au moins un trait qui s'écarte de la population de contrôle, la méthode comprenant:
a) l'obtention des mesures de traits qui déterminent un stade de croissance pour cette population de contrôle et cet organisme candidat de vérification;
b) facultativement, déterminant à quel stade de croissance un organisme se trouve, sur la base de mesures obtenues à l'étape a);
c) déterminant quels traits supplémentaires de l'organisme doivent être mesurés à ce stade de croissance;
d) obtenant des mesures pour les traits ainsi qu'il est déterminé à l'étape c) qui se manifestent ou sont associés à ce stade de croissance pour ce contrôle et ces organismes candidats de vérification;
e) facultativement, répétant les étapes a), b), c) et d) pour mesurer des stades de croissance supplémentaires; et
f) facultativement, identifiant ces organismes candidats de vérifications ayant au moins un trait qui s'écarte de la population de contrôle basée sur ces traits mesurés de stade de croissance et/ou des traits qui peuvent se manifester ou être associés avec au moins un stade de croissance.

4. Un milieu qu'un ordinateur peut lire selon la revendication 3, dans lequel l'organisme est une plante.

5. Un milieu qu'un ordinateur peut lire selon la revendication 3, dans lequel l'organisme est une plante dicotylédone ou monocotylédone.

6. Un milieu qu'un ordinateur peut lire selon la revendication 3, dans lequel l'organisme est une plante dicotylédone.

7. Un milieu qu'un ordinateur peut lire selon la revendication 3, dans lequel le stade de croissance est un ou plusieurs des suivants: germination, pousse des feuilles, croissance des cotylédons, croissance de la rosette, apparition de la fleur, floraison, développement du fruit, mûrissement du fruit, sénescence ou une combinaisons de ces stades.

8. Un milieu qu'un ordinateur peut lire selon la revendication 3, dans lequel les traits de l'organisme à mesurer sont de un à plusieurs des caractéristiques qui suivent: relatifs au développement, de nature morphologique, visuelle, physiologique, moléculaire, biochimique ou des combinaisons de ceux-ci.

9. La méthode de la revendication 2, dans laquelle les mesures de traits sont recueillies en utilisant un ou plusieurs des moyens suivants: photographie macroscopique, mesures physiques, dissection, quantification de couleurs, photographie microscopique, détermination de poids, analyse d'image, mesures de spectroscopie à fluorescence ou des combinaisons de celles-ci.

10. Un milieu qu'un ordinateur peut lire dont le contenu transforme un système d'ordinateur en un système pour identifier un organisme candidat de vérification qui a au moins un trait qui s'écarte d'une population de contrôle, le milieu comprenant:
a) un sous-système qui reçoit des mesures de traits déterminants de stade de croissance;
b) un sous-système optionnel qui détermine à quel stade de croissance se trouve un organisme sur la base de mesures reçues dans le sous-système de l'étape a);
c) un sous-système qui spécifie quel traits de l'organisme sont à mesurer à ce stade de croissance;
d) un sous-système qui reçoit des mesures de traits de l'organisme à mesurer à un stade de croissance pour ces organismes de contrôle et de vérification;
e) un sous-système facultatif qui peut répéter les étapes a), b), c) et d) pour mesurer des stades de croissance supplémentaires; et
f) un sous-système facultatif qui peut identifier ces organismes de candidats de vérifications ayant au moins un trait qui s'écarte de la population de contrôle sur la base de traits de stade de croissance mesurés et/ou de traits qui peuvent être manifestés ou associés à au moins un stade de croissance.

11. Un système d'ordinateur programmé pour identifier un organisme candidat de vérification qui présente au moins un trait qui s'écarte d'une population de contrôle, le système comprenant:
a) un sous-système qui reçoit des mesures de traits déterminant un stade de croissance;
b) un sous-système facultatif qui détermine le stade de croissance auquel se trouve un organisme sur la base de mesures reçues dans le sous-système a);
c) un sous-système qui spécifie quels traits de l'organisme doivent être mesurés à ce stade de croissance;
d) un sous-système qui reçoit des mesures de traits de l'organisme à mesurer à un stade de croissance pour ces organismes candidats et contrôles;
e) un sous-système facultatif qui peut répéter les étapes a), b), c) et d) pour mesurer des stades de croissance supplémentaires; et
f) un sous-système facultatif qui peut identifier ces organismes candidats de vérifications ayant au moins un trait qui s'écarte de la population de contrôle sur la base de traits mesurés de stade de croissance et/ou de traits qui peuvent se manifester ou être associés à au moins un stade de croissance.

12. Une méthode pour identifier un organisme candidat de vérification qui a au moins un trait qui s'écarte d'une population de contrôle, la méthode comprenant:
a) obtenant des mesures de traits qui déterminent un stade de croissance pour cette population de contrôle et cet organisme candidat de vérification;
b) déterminant à quel stade de croissance une organisme se trouve sur la base de mesures obtenues à l'étape a);
c) déterminant quels traits supplémentaires de l'organisme doivent être mesurés à ce stade de croissance;
d) obtenant des mesures pour les traits ainsi qu'il est déterminé à l'étape c) qui se manifestent ou sont associés à ce stade de croissance pour les organismes contrôles et candidats de vérifications;
e) facultativement, répétant les étapes a), b), c) et d) pour mesurer des stades de croissance supplémentaires; et
f) identifiant ces organismes de candidats de vérifications ayant au moins un trait qui s'écarte de la population de contrôle sur la base de ces traits mesurés de stade de croissance et/ou de traits qui peuvent se manifester ou être associés à au moins un stade de croissance.

13. La méthode de la revendication 1, 2 ou 12, dans laquelle l'organisme est une plante.

14. La méthode de la revendication 1, 2 ou 12, dans laquelle l'organisme est une plante dicotylédone ou une plante monocotylédone.

15. La méthode de la revendication 1, 2 ou 12, dans laquelle l'organisme est une plante dicotylédone.

16. La méthode de la revendication 15, dans laquelle le stade de croissance est un ou plusieurs des suivants: germination, apparition des feuilles, croissance du cotylédon, croissance de la rosette, émergence de la fleur, floraison, développement du fruit, sénescence, ou des combinaisons de ceux-ci.

17. La méthode des revendication 1, 2 ou 12, dans laquelle les traits de l'organisme à mesurer sont un ou plusieurs des caractéristiques suivants: relatifs au développement, morphologiques, de nature visuelle, physiologique, moléculaire, biochimique ou des combinaisons de ceux-ci.

18. La méthode de la revendication 1, 2 ou 12, dans laquelle les mesures de traits sont recueillies en utilisant un ou plusieurs des suivants: photographie macroscopique, mesures physiques, dissection, quantification de couleur, photographie microscopique, détermination de poids, analyse de l'image, mesures spectroscopique fluorescents ou une combinaison de ceux-ci.
